# EUROPEAN PATENT APPLICATION

(11) **EP 2 374 813 A1**
(43) Date of publication of application: **12.10.2011**
(21) Application number: 09803779.9
(22) Date of filing: 02.12.2009
(51) Int. Cl.: C07K 14/31

(54) **USE OF PHENOL-SOLUBLE MODULINS FOR VACCINE DEVELOPMENT**

(30) Priority: 03.12.2008 ES 200803441
(71) Applicant: Proyecto de Biomedicina Cima, S.L., 31008 Pamplona - Navarra (ES); Institut Pasteur, 75724 Paris Cedex 15 (FR)
(72) Inventor: BORRÁS CUESTA, Francisco, E-31008 Pamplona - Navarra (ES); CASARES LAGAR, Inés Noelia, E-31008 Pamplona - Navarra (ES); DURÁNTEZ DELGADO, María del Carmen, E-31008 Pamplona - Navarra (ES); LASARTE SAGASTIBELZA, Juan José, E-31008 Pamplona - Navarra (ES); LECLERC, Claude, F-75724 París Cedex 15 (FR); PRIETO VALTUEÑA, Jesús María, E-31008 Pamplona - Navarra (ES); SAROBE UGARRIZA, Pablo, E-31008 Pamplona - Navarra (ES)
(74) Representative: ABG Patentes, S.L.
(86) International application number: PCT/ES2009/070546
(87) International publication number: WO 2010/063865

(57) **Abstract**

The invention relates to methods for increasing immunogenicity of an antigenic peptide by means of its covalent coupling to a modulin derived peptide (PSM, phenol soluble modulin). In particular, the binding of PSMα, PSMγ and PSMδ peptides to an antigen (from a pathogen or a tumor associated protein) increases the capacity of the antigen to activate an immune response in vivo. Thus, the PSMα, PSMγ and PSMδ peptides bound to these antigens may be used in the development of vaccines for preventing or treating infectious diseases or cancer

## Description

### FIELD OF THE INVENTION

The invention generally relates to the field of immunology and, in particular, to methods for increasing the immunogenicity of an antigenic peptide by means of their covalent coupling to a modulin-derived peptide (PSM, phenol soluble modulin). In particular, the binding of the PSMα, PSMγ and PSMδ peptides to an antigen (from a pathogen or a protein associated to a tumor) increases the capacity of the antigen to activate an immune response *in vivo.* Thus, the PSMα, PSMγ and PSMδ peptides bound to these antigens can be used in the development of vaccines for preventing or treating infectious diseases or cancer.

### BACKGROUND OF THE INVENTION

Pathogens and cancer are still the main causes of death in the world. The development of vaccines for preventing diseases for which there is no vaccination -such as AIDS or malaria- or for treating chronic diseases or cancers, as well as the improvement of the efficacy and safety of already existing vaccines, are still a priority. In most cases, the development of such vaccines requires strategies capable of specifically stimulating CD8+ cytotoxic T lymphocytes (CTLs).

CTLs are activated by means of the presentation to T cell receptors (TCRs) of small peptides associated to MHC class I molecules. These peptide-MHC class I complexes are present on the surface of antigen-presenting cells (APCs), which are also capable of providing stimulus signals for the optimal activation of CTLs.

Dendritic cells (DCs) are the most potent APCs and have a unique capacity for interacting with non-activated T lymphocytes (naive T lymphocytes) and initiating the primary immune response, activating CD4+ helper T lymphocytes and CD8+ cytotoxic T lymphocytes.

In the absence of inflammation and of immune response, dendritic cells move through the blood, peripheral tissues, lymph and secondary lymphoid organs. In peripheral tissues, dendritic cells capture own and foreign antigens. The captured antigens are processed to proteolytic peptides and pass to the MHC class I and II molecules (for the activation of CD8+ or CD4+ T lymphocytes, respectively). This process of antigen capture, degradation and loading is called antigen presentation. However, in the absence of stimulation, peripheral dendritic cells present antigens inefficiently. The exogenous signal or signals from the pathogens or the endogenous signal or signals induces or induce dendritic cells so that they initiate a development process called maturation, which transforms dendritic cells into APCs and into T lymphocyte activators.

Bacterial and viral products, as well as inflammatory cytokines and other own molecules, induce the maturation of dendritic cells by means of direct interaction with the surface receptors of innate dendritic cells. T lymphocytes, through CD40-dependent and independent pathways, and endothelial cells contribute to the final maturation of dendritic cells by means of direct cell to cell contact and by means of cytokine secretion. A short time after a danger signal arises, the efficiency of the antigen capture, intracellular transport and degradation, and intracellular traffic of MHC molecules are modified. The peptide load increases, as well as the half-life and the transfer of MHC molecules to the cell surface. The expression on the surface of the T cell co-stimulatory molecules also increases. In this way, dendritic cells are converted into the most potent APCs, and the only ones capable of activating non-activated T lymphocytes and initiating the immune response. Together with the modification of their capacities in antigen presentation, maturation also induces the massive migration of dendritic cells out of the peripheral tissues. The modifications in the expression of chemokine receptors and adhesion molecules, as well as the important changes in the organization of the cytoskeleton, contribute to the migration of dendritic cells through the lymph until the secondary lymphoid organs.

Dendritic cells respond to two types of signals: to the direct recognition of pathogens (by means of receptors with a specific recognition pattern), and to the indirect recognition of infection (by means of inflammatory cytokines, internal cell compounds and specific immune responses). In response to these signals, dendritic cells are activated and initiate their maturation process, which transforms them into efficient T cell stimulators. One of the most efficient signals for the maturation of DCs is mediated by the interactions of toll-like receptors, TLRs, (TLR1-9) with their respective ligands (reviewed by Kaisho and Akira, Biochimica et Biophysica Acta, 2002:1589:1-13).

A first approach to target antigenic peptides to MHC class I and/or II molecules is based on synthetic peptide vaccines containing selected epitopes capable of binding directly to these molecules on the surface of the APCs. In some cases these peptides have achieved tumor protection or virus elimination in murine models, whereas in other cases they have induced tolerance. The studies with different types of peptides in humans have reached timid clinical responses in patients with cancer. This poor immunogenic capacity can be due to the fact that peptides generally do not activate the maturation of dendritic cells, such that antigen presentation occurs in a non-immunogenic environment, the non-response to the antigen (anergy) being favored.

Alternatively, several approaches have been described wherein the peptides are targeted to the APC by the use of compositions wherein the antigenic peptides are provided with a second compound showing affinity towards a receptor on the surface of the APC. In this sense, the use of TLR ligands for targeting an immunogenic peptide to the APC has been described in the past.

TLRs are expressed in macrophages and in dendritic cells, and in other cells such as B lymphocytes. Ligands for several TLRs have also been identified. Most of these ligands come from pathogens but they are not found in the host, suggesting that TLRs are essential for detecting invading microorganisms. The recognition of ligands by TLRs gives rise to a quick activation of the innate immunity upon inducing the production of pro-inflammatory cytokines and to the overregulation of co-stimulatory molecules. The activated innate immunity gives rise to an efficient adaptive immunity.

Different TLR ligands have been used for increasing the efficiency of an antigen such as including EDA (Lasarte et al. J Immunol, 2007, Spanish patent application ES200501412), flagellin (Cuadros C et al., Infect Immun. 2004 May;72:2810-6) or CpGs (Tighe, H., et al. 2000. Eur J Immunol. 30:1939).

WO2005025614 describes adjuvant compositions comprising a nucleic acid encoding a TLR agonist and a nucleic acid encoding GM-CSF, although no mention is done in this document that GM-CSF might be provided as a fusion protein with the TLR agonist.

WO2004060319 describes adjuvant compositions comprising a combination of a TLR agonist and a TNF receptor agonist for increasing the immune response against an antigen but wherein both components are not covalently linked.

WO07042583 describes immunostimulatory composition against hepatitis C virus comprising a TLR3 agonist (poly I:C), a CD40 agonist and a NS3 polypeptide. It does not teach covalent complexes between any of the elements of the composition.

WO06134190 describes immunostimulatory compounds comprising a fibronectin fragment with affinity towards TLR4. This document mentions the possibility of covalent coupling of the fibronectin fragment and an antigen. However, this document does not mention similar compounds using TLR2 agonists or PSM.

WO2007103322 describes fusion proteins comprising an immunogenic polypeptide and either a TLR5 agonist (Flagelin) or a TLR2/6 agonist (the Pam₃Cys lipopeptide) and the uses thereof to induce an antibody response against the immunogenic peptide.

WO2006083706 describes a screening method for the identification of TLR2 ligands and the isolation of a series of new TLR2 ligands as well as covalent conjugates comprising these ligands and an antigen which can be used to generate protective immunisation.

However, there is still a need for further immunogenic compositions which are capable of (i) transporting antigen-derived T lymphocyte epitopes to APCs (or DCs) so that they are loaded in the MHC class I and/or II molecules, (ii) transmit the suitable signals to the DC to induce its activation since the arrival of the antigen to the DC without the existence of a maturation signal could cause tolerance instead of activation of helper and cytotoxic T lymphocytes and (iii) lead to efficient immunogenicity irrespective of prior immunity against the carrier itself.

### SUMMARY OF THE INVENTION

In a first aspect, the invention relates to a conjugate comprising
(i) a phenol-soluble modulin (PSM) or a functionally-equivalent variant thereof and
(ii) one or more antigenic peptides
wherein components (i) and (ii) are covalently coupled and wherein the conjugate promotes a cytotoxic response towards the antigenic peptide or peptides.

In further aspects, the invention relates to a polynucleotide or gene construct comprising a nucleic acid sequence encoding a conjugate of the invention, to a vector comprising a polynucleotide or gene construct of the invention and to a host cell comprising a polynucleotide, a gene construct or a vector of the invention.

In another aspect, the invention relates to a composition comprising, together or separately,
(a) A conjugate, a polynucleotide, a gene construct, a vector or a host cell of the invention and
(b) a second component selected from the group of
   (i) one or more toll-like receptor agonists,
   (ii) one or more agonist antibodies of a co-stimulatory molecule,
   (iii) one of more cytokines and
   (iv) any combination of the compounds mentioned in (i) to (iii)

In another aspect, the invention relates to a method for obtaining an antigen-primed antigen-presenting cell comprising the steps of
(i) contacting an antigen-presenting cell with a conjugate, a polynucleotide, a gene construct, a vector, a host cell or a composition of the invention and
(ii) isolating the antigen-primed antigen-presenting cell.

In another aspect, the invention relates to an antigen-primed antigen-presenting cell obtained by the method of the invention.

In another aspect, the invention relates to a pharmaceutical composition or a vaccine comprising a conjugate, a polynucleotide, a gene construct, a vector, a host cell, a composition or an antigen-primed antigen-presenting cell.

In another aspect, the invention relates to a conjugate, a polynucleotide, a gene construct, a vector, a host cell, a composition, a pharmaceutical composition, a vaccine or an antigen presenting cell of the invention for use in medicine.

In yet another aspect, the invention relates to a conjugate, a polynucleotide, a gene construct, a vector, a host cell, a composition of the invention, a pharmaceutical composition, a vaccine or an antigen presenting cell of the invention for use in a method of inducing a cytotoxic response towards an infectious disease, an allergic disease or a neoplastic disease.

In yet another aspect, the invention relates to a conjugate, a polynucleotide, a gene construct, a vector, a host cell, a composition, a pharmaceutical composition, a vaccine or an antigen presenting cell of the invention for use in an in vitro method for promoting the presentation of the antigenic peptide or peptides by antigen-presenting cells or for promoting maturation of antigen-presenting cells.

In another aspect, the invention relates to a conjugate comprising
(i) a phenol-soluble modulin (PSM) or a functionally-equivalent variant thereof and
(ii) a biologically active compound
wherein components (i) and (ii) are covalently coupled.

In further aspects, the invention relates to a polynucleotide or gene construct comprising a nucleic acid sequence encoding a non-immunogenic conjugate of the invention, a vector comprising a polynucleotide or gene construct of the invention, a host cell comprising a polynucleotide or gene construct of the invention.

In another aspect, the invention relates to a pharmaceutical preparation comprising a non-immunogenic conjugate, a polynucleotide, gene construct, a vector or a host cell of the invention and a pharmaceutically acceptable carrier.

In yet another aspect, the invention relates to a conjugate, a polynucleotide, a gene construct, a vector, a host cell or a pharmaceutical composition of the invention for use in medicine.

In yet another aspect, the invention relates to a non-immunogenic conjugate, a polynucleotide, a gene construct, a vector, a host cell or a pharmaceutical composition of the invention for use in the treatment of a disease characterized by an undesired proliferation or an undesired activity of a cell selected from the group of a CD4-, CD8-, CD 19-, CD11c-, F4/8- or a CD 17-positive cells or a combination thereof.

### BRIEF DESCRIPTION OF THE FIGURES

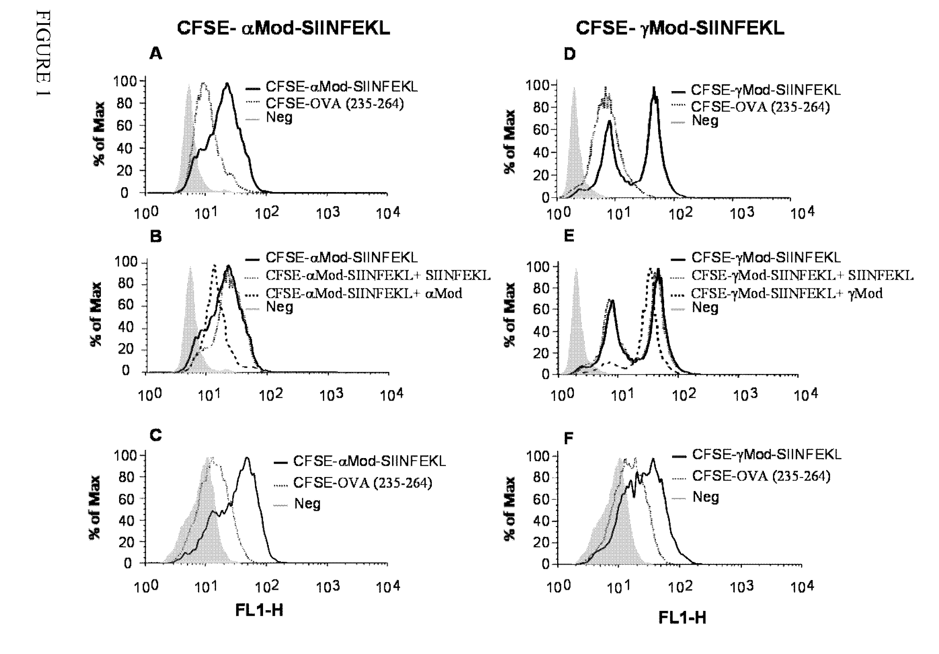

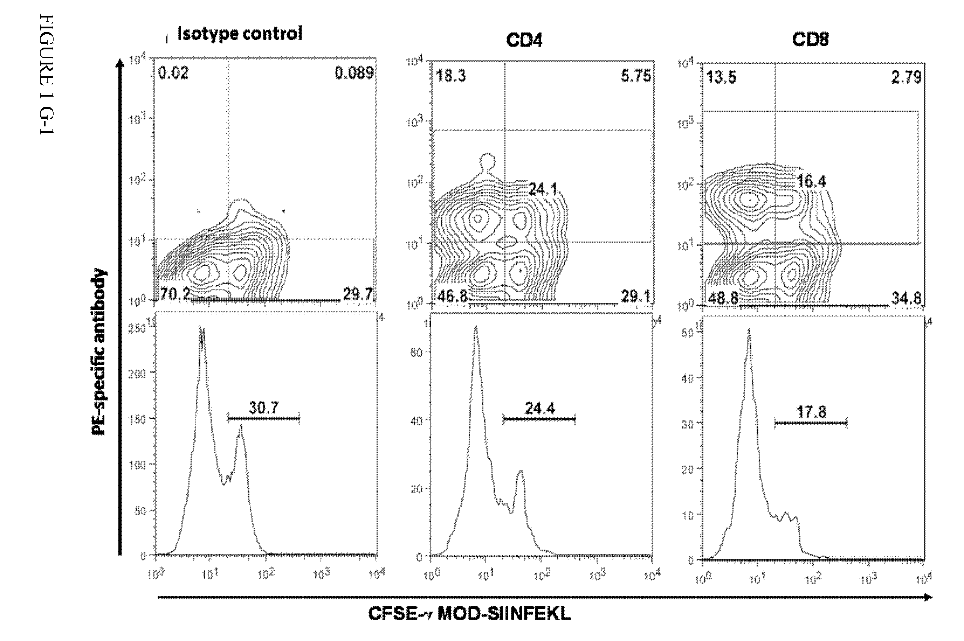

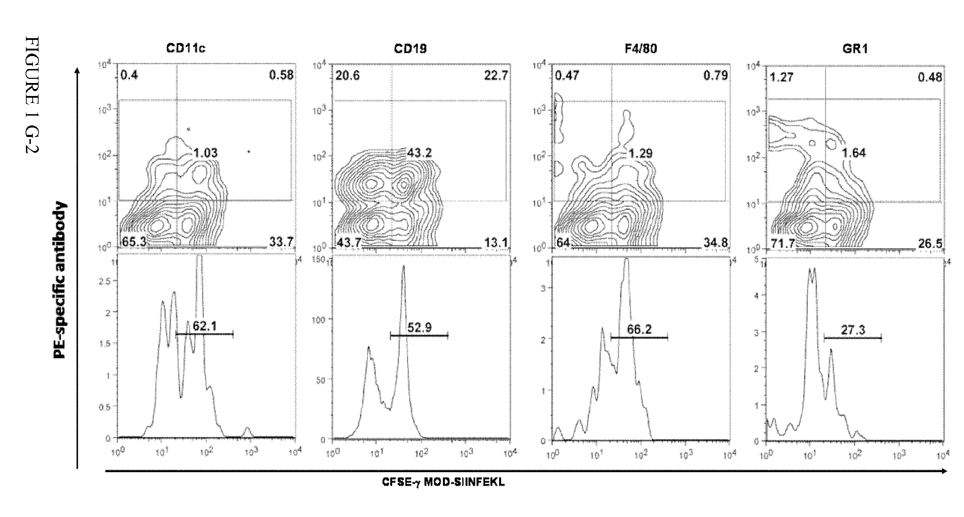

**Figure 1****. PSM peptides bind to mouse splenocytes**. The mouse splenocytes (panels A, B, D and E) or bone marrow derived dendritic cells (panels C and F) were fixed with glutaraldehyde and then incubated with either CFSE αMod-SIINFEKL peptide (panels A, B and C) or with the CFSE γMod-SIINFEKL peptide (panels D, E and F), containing the PSMα or the PSMγ peptide covalently bound to the SIINFEKL peptide and labeled with CFSE at an amino terminal end. As controls, cells were also stained with the labeled control peptide CFSE-OVA(235-264) (A, C, D and F). To study the specificity of binding we carried out the incubation of the CFSE labeled peptides in the presence or absence of αMod-SIINFEKL (B) or γMod-SIINFEKL (D) peptides not labeled with CFSE as competitors. After 30 minutes at 4°C and two washings with PBS, the cells were analyzed by flow cytometry. (G) Double staining of the total splenocytes with the CFSE γMod-SIINFEKL peptide and with anti-CD4, CD8, CD11c, CD19, F480 or GR1antibodies labeled with phycoerythrin. The bottom histograms of panel G indicate the percentage of positive cells for these surface markers which are stained with the CFSE γMod-SIINFEKL peptide.

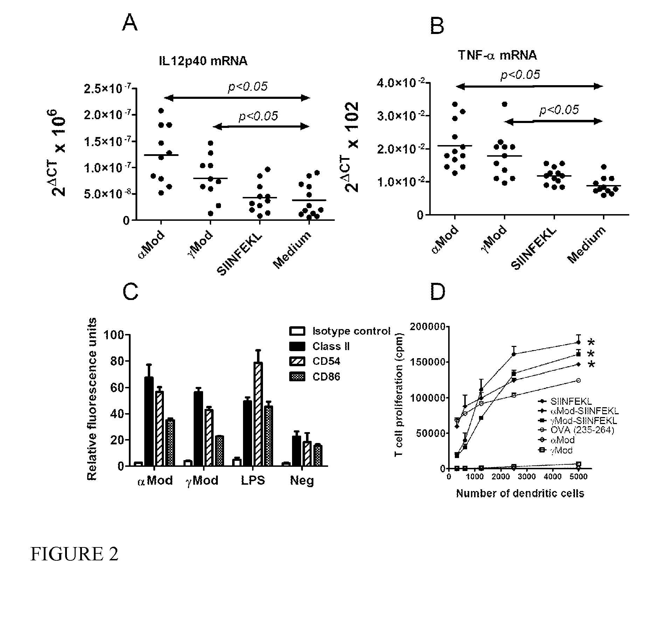

**Figure 2****. PSM peptides induce the maturation of dendritic cells derived from bone marrow and improve antigen presentation.** Expression of messenger RNA encoding IL-12 proteins (p40) (A) or TNF-α (B). The dendritic cells derived from bone marrow were cultured with the indicated peptides at a concentration of 50 µM. After 14 hours of culture, the RNA of the cells was purified and its reverse transcription was carried out. The expression of mRNA for IL12-p40 and TNFα was carried out by real-time PCR. (C) Expression of maturation markers on the cell surface of dendritic cells.. The dendritic cells derived from bone marrow were cultured as described in (A) in the presence of the indicated peptides. After 48 hours of culture, the expression of the indicated markers was analyzed by flow cytometry using specific antibodies. (RFU: Relative Fluorescence Units) (D) *In vitro* assay for antigen presentation. Bone marrow-derived dendritic cells were cultured in the presence or absence of 10 µM of the indicated peptides. Forty hours later, the cells were washed and distributed in 96-well plates at different concentrations and incubated in the presence of 10⁵ T cells/well obtained from transgenic OT-1 mice (which express a specific T receptor for the SIINFEKL peptide of ovalbumin). Seventy-two hours later, tritiated thymidine was added to the cultures and six hours later, the cells were harvested and the incorporated thymidine was analyzed in a scintillation counter (Topcount Packard).

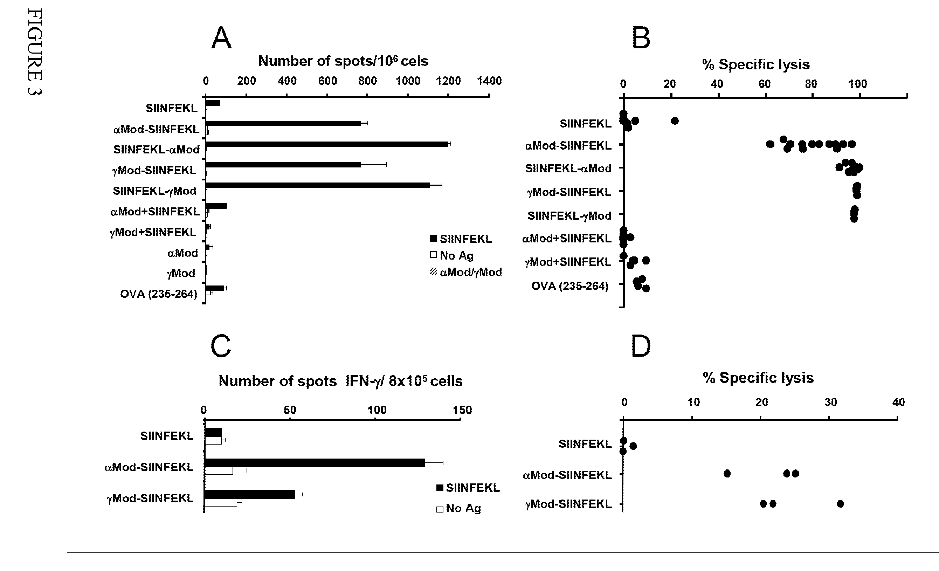

**Figure 3****. Activation of an *in vivo* cell response specific for SIINFEKL peptide.** C57BL/6 were immunized with 5 nmoles of the indicated peptide plus 50 µg of poly I:C. Seven days (A and B) or 60 days (C and D) after immunizations, mice were sacrificed and ELISPOT assay (A and C) was carried out to quantify the number of IFNγ-producing cells in response to the SIINFEKL peptide or the corresponding modulin peptide in each of the groups of immunized mice. (B and D) Measurement of the specific cytotoxic activity against the SIINFEKL peptide induced seven days (B) or 60 days (D) after the immunization with the different peptides. The *in vivo* lysis (in vivo killing) assay was carried out by intravenously injecting the mice with splenocytes labeled with 0.25 µM CFSE or with 2.5 µM CFSE and the SIINFEKL peptide (10 µg/ml). After 16 hours, the mice were sacrificed and the splenocytes were analyzed by flow cytometry to quantify the lysis of the cells pulsed with the SIINFEKL peptide.

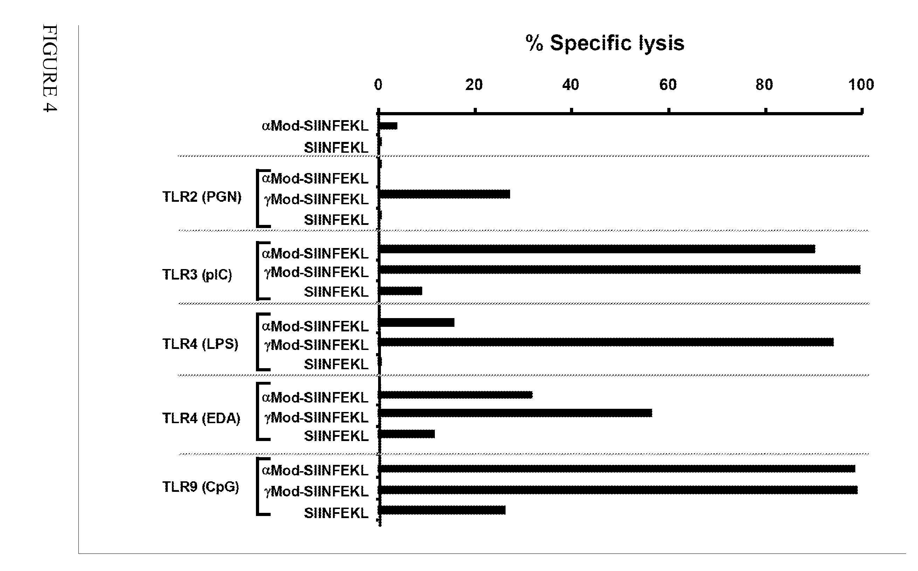

**Figure 4****. Synergy of modulins with different TLR ligands in the in vivo activation of cell responses.** C57BL/6 mice were immunized with 5 nmoles of the indicated peptide and in combination with 50 µg of the indicated TLR ligand: PGN (peptidoglycan), pIC (poly I:C), LPS (lypopolysaccharide), EDA (extra domain A from fibronectin), or CpG. After seven days, the specific cytotoxic activity against the SIINFEKL peptide induced after the immunization with the different peptides was measured. An in vivo lysis (in vivo killing) assay was carried out by intravenously injecting the mice with splenocytes labeled with 0.25 µM CFSE or with 2.5 µM CFSE and the SIINFEKL peptide (10 µg/ml). After 16 hours, the mice were sacrificed and the splenocytes were analyzed by flow cytometry to quantify the lysis of the cells pulsed with the SIINFEKL peptide.

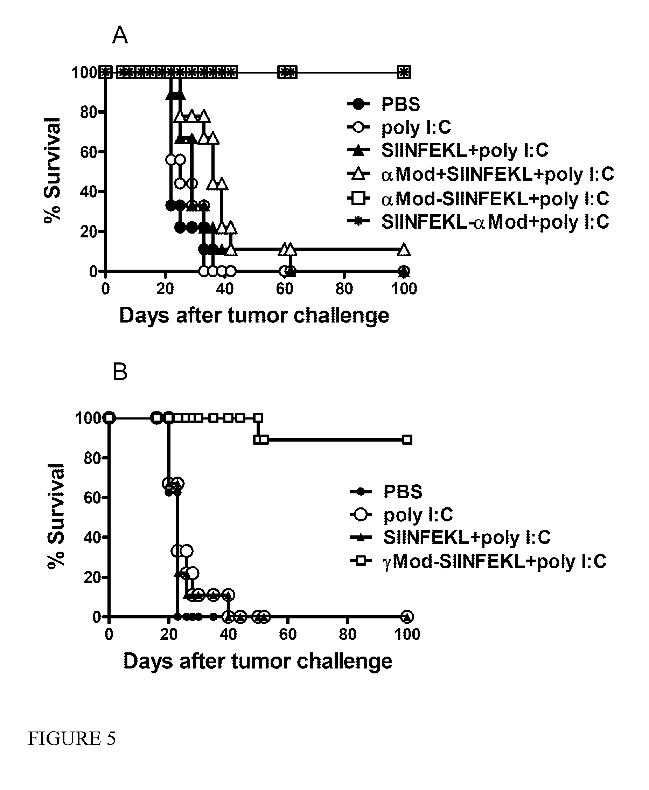

**Figure 5****. Protection against the development of tumors.** (A) C57BL/6 mice were subcutaneously immunized intravenously with 5 nmoles of SIINFEKL peptide, αMod plus SIINFEKL (not covalently linked), αMod-SIINFEKL or SIINFEKL-αMod, all of them in combination with 50 µg of poly I:C. A group of mice received poly I:C alone and another group was injected with PBS (as a tumor growth control). (B) C57BL/6 mice were subcutaneously immunized intravenously with 5 nmoles of SIINFEKL peptide or with γMod-SIINFEKL peptide in combination with poly I:C. A group of mice received poly I:C alone and another group was injected with PBS (as a tumor growth control). Seven days after the immunization, 5x10⁻⁵ (A) or 7x10⁵ (B) EG7OVA tumor cells were subcutaneously injected. The follow-up of the tumor growth was monitored with a gage. Kaplan-Meier plot of mice survival for each treatment is depicted. Effect of αMod-SIINFEKL (A) and γMod-SIINFEKL (B) on protection against tumor challenge.

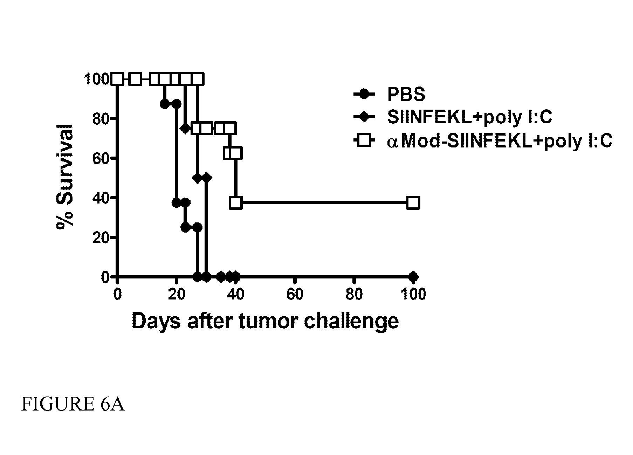

**Figure 6A****.** Treatment of mice bearing EG7OVA established tumors with peptide αMod-SIINFEKL peptide plus poly I:C. C57BL/6 mice were injected s.c with 5x10⁵ EG7OVA tumor cells. When tumors reached 5 mm in diameter they were treated with PBS or with the indicated peptide plus poly I:C. The follow-up of the tumor growth was monitored with a gage. Kaplan-Meier plot of mice survival for each treatment is depicted.

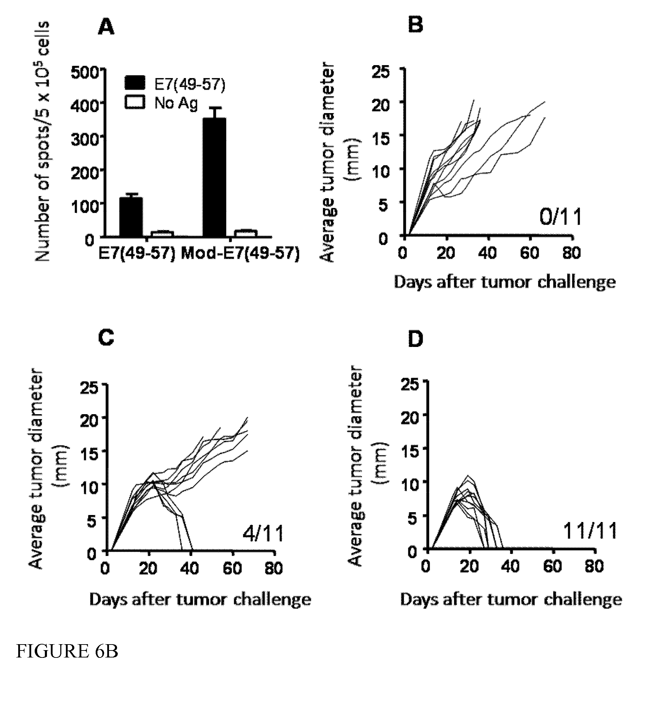

**Figure 6B****. Immunization with αMod-E7 (49-57) peptide in combination with poly I:C induces a specific cytotoxic response against the E7 (49-57) peptide and is capable of curing mice carrying TC1 subcutaneous tumors.** (A) C57B/6 mice were immunized with 5 nmoles of the indicated peptide in combination with 50 µg of poly I:C. Seven days after immunization, the induced response against the cytotoxic peptide E7 (49-57) was measured with ELISPOT to quantify the number of cells producing IFN-γ. (B), (C) and (D) C57B/6 mice were subcutaneously injected with 5 x 10⁵ TC-1 tumor cells (expressing HPV-16 E7 protein). After 25 days, when the tumor had 8 mm diameter, mice were intravenously treated with PBS (B), with E7 (49-57) peptide + 50 µg/ml poly I:C (C) or with αMod-E7 (49-57) peptide + 50 µg/ml poly I:C (D). After seven days, mice were immunized again with a second immunization with the same immunogens. Tumor size, presented as the average of two perpendicular diameters, was measured regularly. The number of tumor-free mice relative to the number of total mice per group is included for each treatment.

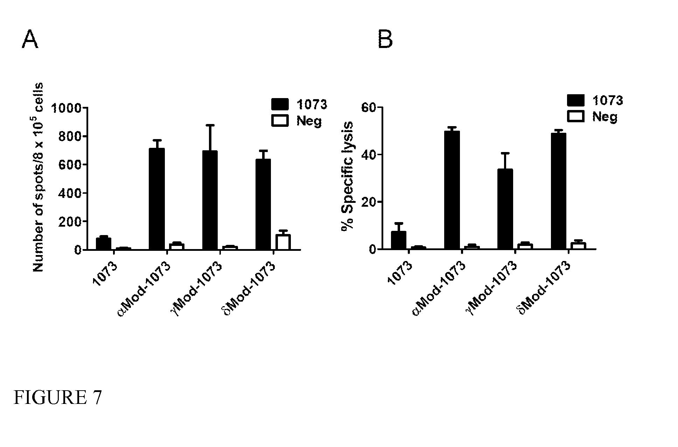

**Figure 7****. In vivo induction of T cell responses against the hepatitis C NS3 peptide p1073 by using α, γ or δ-derived modulin peptides coupled to p1073.** C57BL/6 were immunized with 5 nmoles of the indicated peptide plus 50 µg of poly I:C. Seven days later, mice were sacrificed and ELISPOT assay (A) was carried out to measure the presence of IFNγ producing cells specific for peptide p1073 (1073) from hepatitis C NS3 protein. In vivo killing assay (B) was performed using spleen cells labeled with 0.25 µM CFSE or with 2.5 µM CFSE and peptide p1073 (10 µg/ml).

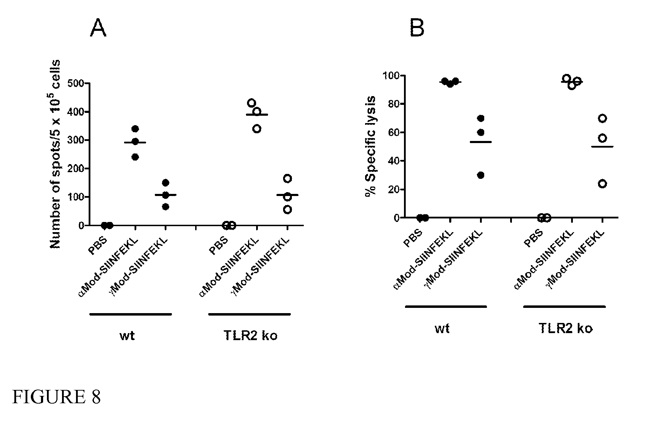

**Figure 8****. Activation of a cell response induced by modulin-derived peptides in KO mice for TLR2.** C57BL/6 wild type mice or TLR2 KO mice were immunized with 5 nmoles of the indicated peptide and in combination with 50 µg of poly I:C. Seven days after the immunization, the animals were sacrificed and an ELISPOT was carried out to quantify the number of IFNγ producing cells in response to the SIINFEKL peptide. (B) Measurement of the specific cytotoxic activity against the SIINFEKL peptide induced after the immunization with the different peptides. An *in vivo* lysis (*in vivo* killing) assay was carried out by intravenously injecting mice with splenocytes labeled with 0.25 µM C mM CFSE or with 2.5 µM CFSE and the SIINFEKL peptide (10 µg/ml). After 16 hours, the mice were sacrificed and the splenocytes were analyzed by flow cytometry to quantify the lysis of the cells pulsed with the SIINFEKL peptide.

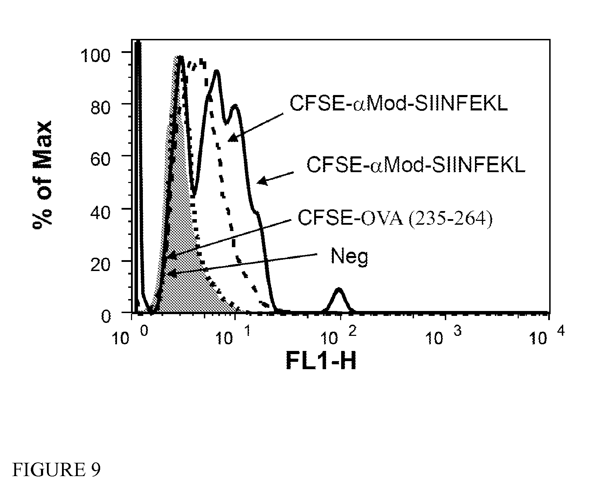

**Figure 9****. PSM peptides bind to mouse splenocytes from TLR2 knock out mice.** Mouse splenocytes from TLR2 KO mice were fixed with glutaraldehyde and then incubated with either CFSE αMod-SIINFEKL peptide or with the CFSE γMod-SIINFEKL peptide. As controls, cells were also stained with the labeled control peptide CFSE-OVA(235-264) or left untreated (dashed gray histogram). After 30 minutes of incubation at 4°C and two washings with PBS, the cells were analyzed by flow cytometry.

### DESCRIPTION

The authors of the present invention have shown that PSM peptides are capable of carrying an antigen to antigen-presenting cells, activate its maturation, improve the antigen presentation and consequently promote the activation of a immune cell response against the antigen. This strategy has been proved to be effective to induce cytotoxic responses and to protect C57BL/6 mice from the development of tumors. It has been observed, however, that the mechanism of action of the peptides derived from modulin is independent of TLR2. In fact, the immunization of mice with the peptides used, which contain alpha or gamma modulins bound to an antigen, induces strong cell responses even in knockout mice for the TLR2 receptor.

### I. THE CONJUGATES OF THE INVENTION

The authors of the present invention have shown that covalent conjugates comprising a phenol-soluble modulin and an antigenic peptide are capable of promoting maturation of dendritic cells and peptide presentation by dendritic cells of peptides covalently coupled to said PSMs.

As shown in example 2 of the present invention, fusion proteins comprising a PSM and a peptide promote maturation of dendritic cells and antigen presentation to a higher degree than that observed with the antigenic peptide alone. Moreover, example 3 of the invention shows that the administration of the conjugates leads to a strong and long term cytotoxic response which is not observed after the administration of a composition comprising a non covalent mixture of the PSM and the antigenic peptide.

Thus, in a first aspect, the invention relates to a conjugate comprising
(i) a phenol-soluble modulin (PSM) or a functionally-equivalent variant thereof and
(ii) one or more antigenic peptides
wherein components (i) and (ii) are covalently coupled and wherein the conjugate promotes a cytotoxic response towards the antigenic peptide or peptides.

### A. The phenol soluble modulin

The first component of the conjugates of the invention corresponds to a phenol-soluble modulin or a functionally equivalent variant thereof. The terms "phenol-soluble modulin" and "PSM" are used here indistinctly and relate to any member of the family of cytotoxic peptides secreted by different Staphylococcus strains, in particular *Staphylococcus aureus* and *Staphylococcus epidermidis,* and characterized by their ability to partition into the organic phase when extracted with hot phenol.

<223>  MMP-9 cleavage site

<400>  41

Leu Phe Pro Thr Ser
1               5


<210>  42
<211>  45
<212>  PRT
<213>  Artificial

<220>
<223>  PSM fusion

<400>  42

Leu Met Ser Cys Leu Ile Leu Arg Ile Phe Ile Leu Ile Lys Glu Gly
1               5                   10                  15


Val Ile Ser Met Ala Gln Asp Ile Ile Ser Thr Ile Gly Asp Leu Val
            20                  25                  30


Lys Trp Ile Ile Asp Thr Val Asn Lys Phe Thr Lys Lys
        35                  40                  45


<210>  43
<211>  36
<212>  PRT
<213>  Artificial

<220>
<223>  PSM fusion

<400>  43

Met Ala Gln Asp Ile Ile Ser Thr Ile Gly Asp Leu Val Lys Trp Ile
1               5                   10                  15


Ile Asp Thr Val Asn Lys Phe Thr Lys Lys Lys Lys Lys Lys Lys Lys
            20                  25                  30


Lys Lys Lys Lys
        35


<210>  44
<211>  8
<212>  PRT
<213>  Artificial

**Table 1: Phenol-soluble modulins**

| **Organism** | **Name** | **Accession** | **SEQ ID** | **Sequence** |
|---|---|---|---|---|
| S. epidermidis RP62A | PSM alpha | YP_187680 | 1 | MADVIAKIVEIVKGLIDQFTQK |
| S. epidermidis RP62A | PSM beta 1 | YP_188322 | 2 | MSKLAEAIANTVKAAQDQDWTKLGTSIVDIVESGVSVLGKIFGF |
| S. epidermidis RP62A | PSM beta 1 | YP_188320 | 3 | MEQLFDAIRSVVDAGINQDWSQLASGIAGIVENGISVISKLLGQ |
| S. epidermidis RP62A | PSM beta 1 | YP_188319 | 4 | MELLTHLGVLIMKLFNAFKDILEAAITNDGTQLGASIVNI IESSVDMVNRFLGN |
| S. epidermidis RP62A | PSM beta 1 | YP_189947 | 5 | MEHVSKLAEAIANTVSAAQAEDGAELAKSIVNIVANAGGIIQDIAHAFGY |
| S. epidermidis RP62A | PSM beta 1 | YP_189944 | 6 | MEHVSKLGEAIVDTVTAAQAEDGAELAKSIVNIVANAGGIIQDIAHAFGY |
| S.aureus subsp. aureus COL | anti protein | YP_186050 | 7 | MTGLAEAIANTVQAAQQHDSVKLGTSIVDIVANGVGLLGKLFGF |
| S.aureus subsp. aureus COL | anti protein | YP_186049 | 8 | MEGLFNAIKDTVTAAINNDGAKLGTSIVSIVENGVGLLGKLFGF |
| Staphylococcus aureus subsp. aureus MSSA476 | PSM alpha 4 | PSMA4_STAAS | 9 | MAIVGTIIKIIKAIIDIFAK |
| Staphylococcus aureus subsp. aureus MSSA476 | PSM alpha 3 | PSMA3_STAAS | 10 | MEFVAKLFKFFKDLLGKFLGNN |
| Staphylococcus aureus subsp. aureus MSSA476 | PSM alpha 2 | PSMA2_STAAS | 11 | MGIIAGIIKFIKGLIEKFTGK |
| Staphylococcus aureus subsp. aureus MSSA476 | PSM alpha 1 | PSMA1_STAAS | 12 | MGIIAGIIKVIKSLIEQFTGK |
| S.epidermidis | PSMγ | | 13 | MAADIISTIGDLVKWIIDTVNKFKK |
| S.aureus | PSM δ | | 14 | MSIVSTIIEVVKTIVDIVKKFKK |

Most preferably, the PSM used in the conjugates of the invention include the α-, γ- and δ-modulins as described in SEQ ID NO:1, 13 and 14, respectively.

The expression "functionally equivalent variant" of a PSM refers to compounds showing substantially the same biological activity(ies) as PSM. Suitable functional assays to determine whether a given compound is a functionally equivalent variant of the PSM are, e.g. the assay based on the ability of PSM to activate HIV-1 LTR in THP-1 cells as well as the ability of PSM to promote TNF-α secretion by THP-1 cells, both described by Mehlin et al. (J.Exp.Med., 1999, 189:907-917), the assay based on the capacity of promoting NF-kappaB activation in cells expressing TLR2 (either cells transiently transfected with TLR2 or cells which express TLR2 constitutively) as described by Hajjar et al. (Journal of Immunology, 2001, 166:15-19).

Related molecules suitable as component (i) of the conjugates of the present invention also include those sequences which are substantially homologous to the PSM mentioned. The expression "substantially homologous", as used herein, relates to any of the nucleotide sequences describe above when its nucleotide sequence has a degree of identity with respect to the nucleotide sequence of the invention of at least 60%, advantageously of at least 70%, preferably of at least 85%, and more preferably of at least 95%. A nucleotide sequence that is substantially homologous to the nucleotide sequence of the invention can typically be isolated from a producer organism of the polypeptide of the invention based on the information contained in said nucleotide sequence, or it is constructed based on the DNA sequence shown in SEQ ID NO: 1-14, by means of introducing conservative or non-conservative substitutions, for example. Other examples of possible modifications include the insertion of one or more nucleotides in the sequence, the addition of one or more nucleotides in any of the ends of the sequence, or the deletion of one or more nucleotides in any end or inside the sequence. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215: 403-410 (1990)].

Suitable peptides having a substantial sequence homology correspond are shown in table 2. Preferred PSM homologs used in the present invention include the delta-lysins and delta-hemolysins isolated from *Staphylococcus aureus.*

In a preferred embodiment, component (i) of the conjugates of the invention is a PSM selected from the group of SEQ ID NO: 1 to 26.

**Table II: Cytotoxic peptides showing high degree of sequence similarity to the PSMs.**

| **Organism** | **Name** | **Accession** | **SEQ ID** | **Sequence** |
|---|---|---|---|---|
| *S.aureus* | delta-hemolysin | AAF43204 | 15 | MAQDIISTIGDLVKWIIDTVNKFTK |
| *S.aureus* | delta-hemolysin | AAG03054 | 16 | MAQDIISTISDLVKWIIDTVNKFTK |
| *S.intermedius* | delta-hemolysin | CAA11542 | 17 | MAGDIVGTIGEFVKLIIETVQKF |
| *S.hemolyticus* | Antigonococcal protein 1 | P11697 | 18 | MQKLAEAIAAAVQAGQDKDWGKMGTSIVGIVENGISVLGKIFGF |
| *S.hemolyticus* | Antigonococcal protein 2 | P11698 | 19 | MEKIANAVKSAIEAGQNQDWTKLGTSILDIVSNGVTELSKIFGF |
| *S.hemolyticus* | Antigonococcal protein 3 | P11699 | 20 | MSKLVQAISDAVQAQQNQDWAKLGTSIVGIVENGVGILGKLFGF |
| *S.lugdunensis* | SLUSH A | AAB49286 | 21 | MSGIVDAITKAVQAGLDKDWATMATSIADAIAKGVDFIAGFFN |
| *S.lugdunensis* | SLUSH B | AAB49287 | 22 | MSGIIEAITKAVQAGLDKDWATMGTSIAEALAKGIDAISGLFG |
| *S.lugdunensis* | SLUSH C | AAB49288 | 23 | MDGIFEAISKAVQAGLQKDWATMGTSIAEALAKGVDFIIGLGH |
| *S.warneri* | delta-lysin | CAA11542 | 24 | MAGDIVGTIGEFVKLIIETVQKF |
| *S.warneri* | delta-lysin I | CAA11543 | 25 | MAADIISTIGDLVKLIINTVKKFQK |
| *S.warneri* | delta-lysin II | CAA11544 | 26 | MTADIISTIGDFVKWILDTVKKFTK |

### B. The antigenic peptide(s)

Component (ii) of the conjugates of the invention is one or more antigenic peptides. The expression "antigenic peptide", as used herein, is understood as a peptide that stimulates the immune system of a mammal having a tumor or infectious diseases to attack the tumor and inhibits its growth or destroy the pathogen causing the disease. Thus, the antigen used in the invention is matched to the specific disease found in the animal being treated.

In general, the peptide antigen is heterologous to the PSM; that is to say, it is not the PSM protein itself or a fragment thereof, although in certain circumstances it may be desirable to use a fusion protein comprising two copies of the same PSM, e.g. in order to induce a response to the PSM protein itself. The antigen may be derived from the same organism as the PSM. When the antigen is from a species which produces a PSM, it may be desirable to use the PSM from that species, as any immune response which is generated against the PSM itself will contribute to the protection afforded against that organism. For example, when the antigen is from *S. aureus,* the PSM (e.g. the alpha modulin) is preferably also from *S. aureus.*

It will be appreciated that the antigen or antigens may be complete proteins, isolated domains of a protein, peptide fragments of a protein or polyepitopic fusion proteins comprising multiple epitopes (e.g. from 5 to 100 different epitopes). The polypeptide may optionally include additional segments, e.g., it can include at least 4, 5, 10, 15, 20, 25, 30, 40, 50, 60, 75, 90, or even 100 or more segments, each being a portion of a naturally-occurring protein of a pathogenic agent and/or of a naturally occurring tumor antigen which can be the same or different from the protein(s) from which the other segments are derived. Each of these segments can be at least 8 amino acids in length, and each contains at least one epitope (preferably two or more) different from the epitopes of the other segments. At least one (preferably at least two or three) of the segments in the hybrid polypeptide may contain, e.g., 3, 4, 5, 6, 7, or even 10 or more epitopes, particularly class I or class II MHC-binding epitopes. Two, three, or more of the segments can be contiguous in the hybrid polypeptide: i.e., they are joined end-to-end, with no spacer between them. Alternatively, any two adjacent segments can be linked by a spacer amino acid or spacer peptide.

The antigen can be, for example, but is not limited to, a viral antigen, a bacterial antigen, a fungal antigen, a differentiation antigen, a tumor antigen, an embryonic antigen, an antigen of oncogenes and mutated tumor-suppressor genes, a unique tumor antigen resulting from chromosomal translocations and/or derivatives thereof.

### Viral antigens

Viral antigens which are capable of eliciting an immune response against the virus include HIV-1 antigens, (such as tat, nef, gp120 or gp160, gp40, p24, gag, env, vif, vpr, vpu, rev), human herpes viruses, (such as gH, gL gM gB gC gK gE or gD or derivatives thereof or Immediate Early protein such as ICP27 , ICP47, ICP4, ICP36 from HSVl or HSV2, cytomegalovirus, especially Human, (such as gB or derivatives thereof), Epstein Barr virus (such as gp350 or derivatives thereof), Varicella Zoster Virus (such as gpl, II, III and IE63), or from a hepatitis virus such as hepatitis B virus (for example Hepatitis B Surface antigen or Hepatitis core antigen), hepatitis C virus (for example core, E1 NS3 or NS5 antigens), from paramyxoviruses such as Respiratory Syncytial virus (such as F and G proteins or derivatives thereof), from parainfluenza virus, from rubella virus (such as proteins E1 and E2), measles virus, mumps virus, human papilloma viruses (for example HPV6, 11, 16, 18, eg LI, L2, E1, E2, E3, E4, E5, E6, E7), flaviviruses (e.g. Yellow Fever Virus, Dengue Virus, Tick-borne encephalitis virus, Japanese Encephalitis Virus) or Influenza virus cells, such as HA, NP, NA, or M proteins, or combinations thereof), rotavirus antigens (such as VP7sc and other rotaviral components), and the like (see Fundamental Virology, Second Edition, eds. Fields, B. N. and Knipe, D. M. (Raven Press, New York, 1991) for additional examples of viral antigens)

In a preferred embodiment, the antigenic peptide derives from hepatitis C, in a more preferred embodiment, the peptide derives from the hepatitis C NS3 protein. In a more preferred embodiment, the antigenic peptide corresponds to the amino acids 1073-1081 of the HLA-A2-restricted NS3 peptide, whose sequence is CVNGVCWTV (SEQ ID NO:50).

In a preferred embodiment, the antigenic peptide derives from human papillomavirus, in a more preferred embodiment, from human papillomavirus 16, in a more preferred embodiment, the peptide derives from human papillomavirus E7 protein and, in a more preferred embodiment, said peptide comprises the sequence RAHYNIVTF (SEQ ID NO:55).

### Bacterial antigens

The invention contemplates the use of bacterial antigens such as antigens from *Neisseria spp,* including *N. gonorrhea* and *N. meningitidis* (transferrin-binding proteins, lactoferrin binding proteins, PiIC and adhesins); antigens from *S*. *pyogenes* (such as M proteins or fragments thereof and C5A protease); antigens from *S*. *agalactiae, S. mutans; H. ducreyi; Moraxella spp, including M catarrhalis,* also known as *Branhamella* catarrhalis (such as high and low molecular weight adhesins and invasins); antigens from *Bordetella spp,* including *B. pertussis* (for example *parapertussis and B. bronchiseptica* (such as pertactin, pertussis toxin or derivatives thereof, filamenteous hemagglutinin, adenylate cyclase, fimbriae); antigens from *Mycobacterium spp., including M. tuberculosis, M. bovis, M. leprae, M. avium, M. paratuberculosis, M. smegmatis; Legionella spp,* including *L. pneumophila;* (for example ESAT6, Antigen 85A, -B or -C, MPT 44, MPT59, MPT45, HSPIO,HSP65, HSP70, HSP 75, HSP90, PPD 19kDa [Rv3763], PPD 38kDa [Rv0934] ); antigens from *Escherichia spp,* including *enterotoxic E. coli* (for example colonization factors, heat-labile toxin or derivatives thereof, heat-stable toxin or derivatives thereof), antigens from enterohemorragic *E. coli* and enteropathogenic *E. coli* (for example shiga toxin-like toxin or derivatives thereof); antigens from *Vibrio spp,* including *h cholera* (for example cholera toxin or derivatives thereof); antigens from *Shigella spp,* including *S*. *sonnei, S. dysenteriae, S. flexnerii; Yersinia spp,* including Y. enterocolitica (for example a Yop protein); antigens from *7. pestis, Y pseudo tuberculosis; Campylobacter spp,* including *C*. *jejuni* (for example toxins, adhesins and invasins); antigens from *Salmonella spp,* including *S*. *typhi, S. paratyphi, S. choleraesuis, S. enteritidis; Wisteria spp.,* including *L. monocytogenes; Helicobacter spp,* including *H*. pylori (for example urease, catalase, vacuolating toxin); antigens from *Pseudomonas spp,* including *P*. *aeruginosa; Staphylococcus spp.,* including *S*. *aureus, S. epidermidis; Enterococcus spp.,* including *E. faecalis, E. faecium; Clostridium spp.,* including *C*. *tetani* (for example tetanus toxin and derivative thereof); antigens from *C*. *botulinum* (for example botulinum toxin and derivative thereof), antigens from *C*. *difficile* (for example clostridium toxins A or B and derivatives thereof); antigens from *Bacillus spp.,* including *B. anthracis* (for example anthrax toxin and derivatives thereof); *Corynebacterium spp.,* including *C*. *diphtheriae* (for example diphtheria toxin and derivatives thereof); antigens from *Borrelia spp.,* including *B. burgdorferi* (for example OspA, OspC, DbpA, DbpB); antigens from *B. garinii* (for example OspA, OspC, DbpA, DbpB), *B. afzelii* (for example OspA, OspC, DbpA, DbpB), antigens from *B. andersonfi* (for example OspA, OspC, DbpA, DbpB), antigens from *B. hermsii; Ehrlichia spp.,* including *E. equi* and the agent of the Human Granulocytic Ehrlichiosis; *Rickettsia spp,* including *R. rickettsii; Chlamydia spp.,* including *C*. *trachomatis* (for example MOMP, heparin-binding proteins); antigens from *Chlamydia pneumoniae* (for example MOMP, heparin-binding proteins), antigens from *C*. *psittaci; Leptospira spp.,* including *L*. *interrogans; Treponema spp.,* including *T. pallidum* (for example the rare outer membrane proteins), antigens from *T. denticola, T. hyodysenteriae;* antigens from *Plasmodium spp.,* including *P. falciparum; Toxoplasma spp.* and *T. gondii* (for example SAG2, SAGS, Tg34); antigens from *Entamoeba spp.,* including *E. histolytica; Babesia spp.,* including *B. microti; Trypanosoma spp.,* including T. *cruzi; Giardia spp.,* including *G*. *lamblia; leishmania spp.,* including *L. major; Pneumocystis spp.,* including *P. carinii; Trichomonas spp.,* including T. *vaginalis; Schisostoma spp.,* including S. *mansoni,* or derived from yeast such as *Candida spp.,* including *C*. *albicans; Cryptococcus spp.,* including *C*. *neoformans;* antigens from *M. tuberculosis* (such as Rv2557, Rv2558, RPFs: Rv0837c, Rv1884c, Rv2389c, Rv2450, Rv1009, aceA (Rv0467), PstS1, (Rv0932), SodA (Rv3846), Rv2031c 16kDa1., Tb Ra12, Tb H9, Tb Ra35, Tb38-1, Erd 14, DPV, MTI, MSL, mTTC2 and hTCC1); antigens from Chlamydia (such as the High Molecular Weight Protein (HWMP), ORF3 (EP 366 412), and putative membrane proteins (Pmps); antigens from *Streptococcus spp,* including *S*. *pneumoniae* (PsaA, PspA, streptolysin, choline-binding proteins, the protein antigen Pneumolysin, and mutant detoxified derivatives thereof); antigens derived from *Haemophilus spp., including H. influenzae type B* (for example PRP and conjugates thereof); antigens from non typeable *H. influenzae* (such as OMP26, high molecular weight adhesins, P5, P6, protein D and lipoprotein D, and fimbrin and fimbrin derived peptides, or multiple copy variants or fusion proteins thereof); antigens derived from *Plasmodium falciparum* (such as RTS.S, TRAP, MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.)

### Fungal antigens

Fungal antigens for use with the conjugates and methods of the invention include, but are not limited to, e.g., Candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.

### Protozoal and other parasitic antigens

Protozoal antigens include, but are not limited to, *Plasmodium falciparum* antigens such as merozoite surface antigens, sporozoite surface antigens, circumsporozoite antigens, gametocyte/gamete surface antigens, blood-stage antigen pf, 55/RESA and other plasmodial antigen components; toxoplasma antigens such as SAG-1, p30 and other toxoplasmal antigen components; schistosomae antigens such as glutathione- S-transferase, paramyosin, and other schistosomal antigen components; leishmania major and other leishmaniae antigens such as gp63, lipophosphoglycan and its associated protein and other leishmanial antigen components; and *Trypanosoma cruzi* antigens such as the 75-77 kDa antigen, the 56 kDa antigen and other trypanosomal antigen components.

### Allergens and environmental antigens

The antigen can be an allergen or environmental antigen, such as, but not limited to, an antigen derived from naturally occurring allergens such as pollen allergens (tree-, herb, weed-, and grass pollen allergens), insect allergens (inhalant, saliva and venom allergens), animal hair and dandruff allergens, and food allergens. Important pollen allergens from trees, grasses and herbs originate from the taxonomic orders of Fagales, Oleales, Pinoles and platanaceae including La. birch (Betula), alder (Alnus), hazel (Corylus), hornbeam (Carpinus) and olive (Olea), cedar (Cryptomeriaand Juniperus), Plane tree (Platanus), the order of Poales including i.e. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other allergen antigens that may be used include allergens from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, those from mammals such as cat, dog and horse, birds, venom allergens including such originating from stinging or biting insects such as those from the taxonomic order of Hymenoptera including bees (superfamily Apidae), wasps and ants (superfamily Formicoidae). Still other allergen antigens that may be used include inhalation allergens from fungi such as from the genera Alternaria and Cladosporium.

### Tumor antigens

Examples of tumor antigens include MAGE, MART-1/Melan-A, gp100, Dipeptidyl peptidase IV (DPPIV), adenosine deaminase-binding protein (ADAbp), cyclophilin b, Colorectal associated antigen (CRC)-0017-1A/GA733, Carcinoembryonic Antigen (CEA) and its antigenic epitopes CAP-1 and CAP-2, etv6, am11, Prostate Specific Antigen (PSA) and its antigenic epitopes PSA-1, PSA-2, and PSA-3, prostate-specific membrane antigen (PSMA), T-cell receptor/CD3-ζ chain, MAGE-family of tumor antigens (e.g., MAGE-A1, MAGE-A2, MAGE-A3, MAGEA4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGEC5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin,13-catenin, γ-catenin, p120ctn, gp100^{Pme1117}, PRAME, NY-ESO-1, cdc27, adenomatous polyposis coli protein (APC), fodrin, Connexin 37, Ig- idiotype, p15, gp75, GM2 and GD2 gangliosides, viral products such as human papilloma virus proteins, Smad family of tumor antigens, Imp-1, P1A, EBV-encoded nuclear antigen (EBNA)-1, brain glycogen phosphorylase, SSX-1, SSX-2 (HOM-MEL40), SSX-3, SSX-4, SSX-5, SCP-1 and CT-7, and c-erbB-2, acute lymphoblastic leukemia (etv6, amll, cyclophilin b), B cell lymphoma (Ig-idiotype), glioma (E-cadherin, α-catenin,13-catenin, 7-catenin, p120ctn), bladder cancer (p21ras), biliary cancer (p21ras), breast cancer (MUC family, HER2/neu, c-erbB-2), cervical carcinoma (p53, p21ras), colon carcinoma (p21ras, HER2/neu, c-erbB-2, MUC family), colorectal cancer (Colorectal associated antigen (CRC)-0017-1A/GA733, APC), choriocarcinoma (CEA), epithelial cell cancer (cyclophilin b), gastric cancer (HER2/neu, c-erbB-2, ga733 glycoprotein), hepatocellular cancer, Hodgkins lymphoma (lmp-1, EBNA-1), lung cancer (CEA, MAGE-3, NY-ESO-1), lymphoid cell-derived leukemia (cyclophilin b), melanoma (p15 protein, gp75, oncofetal antigen, GM2 and GD2 gangliosides, MelanA/MART-1, cdc27, MAGE-3, p21ras, gp100^{Pme1117}), myeloma (MUC family, p21ras), non-small cell lung carcinoma (HER2/neu, c-erbB-2), nasopharyngeal cancer (lmp-1, EBNA-1), ovarian cancer (MUC family, HER2/neu, c-erbB-2), prostate cancer (Prostate Specific Antigen (PSA) and its antigenic epitopes PSA-1, PSA-2, and PSA-3, PSMA, HER2/neu, c-erbB-2, ga733 glycoprotein), renal cancer (HER2/neu, c-erbB-2), squamous cell cancers of the cervix and esophagus (viral products such as human papilloma virus proteins), testicular cancer (NY-ES0-1), and T cell leukemia (HTLV-1 epitopes).

### The linker connecting components (i) and (ii)

The conjugates of the invention may comprise a linker connecting the first and second component. According to the invention, said non-natural intermediate amino acid sequence acts as a hinge region between domains, allowing them to move independently from one another while they maintain the three-dimensional shape of the individual domains. In this sense, a preferred non-natural intermediate amino acid sequence according to the invention would be a hinge region characterized by a structural ductility allowing this movement. Non-limiting exemplary linkers that can be used to connect components (i) and (ii) of the invention include a non-natural flexible linker. In a preferred embodiment, said flexible linker is a flexible linker peptide with a length of 20 amino acids or less. In a more preferred embodiment, the linker peptide comprises 2 amino acids or more selected from the group consisting of glycine, serine, alanine and threonine. In a preferred embodiment of the invention, said flexible linker is a polyglycine linker. Possible examples of linker/spacer sequences include SGGTSGSTSGTGST (SEQ ID NO:27), AGSSTGSSTGPGSTT (SEQ ID NO:28) or GGSGGAP (SEQ ID NO:29). These sequences have been used for binding designed coiled helixes to other protein domains (Muller, K.M., Arndt, K.M. and Alber, T., Meth. Enzymology, 2000, 328: 261-281). Said linker preferably comprises or consists of the amino acid sequence GGGVEGGG (SEQ ID NO: 30).

The effect of the linker region is providing space between component (i) and component (ii) so that the secondary structure of component (i) is not affected by the presence of component (ii) and vice versa. The spacer preferably has a peptide nature. The linker peptide preferably comprises at least two amino acids, at least three amino acids, at least five amino acids, at least ten amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids or approximately 100 amino acids.

The linker can be bound to components flanking the two components of the conjugates of the invention by means of covalent bonds and preferably the spacer is essentially non-immunogenic and/or does not comprise any cysteine residue. In a similar manner, the three-dimensional structure of the spacer is preferably linear or substantially linear.

Preferred examples of spacer or linker peptides include those which have been used for binding proteins without substantially deteriorating the function of the bound proteins or at least without substantially deteriorating the function of one of the bound proteins. More preferably, the spacers or linkers have been used for binding proteins comprising structures with coiled helixes.

The linker can include residues 53-56 of tetranectin, forming a β sheet in tetranectin, and residues 57-59 forming a turn in the tetranectin (Nielsen, B.B. et al., FEBS Lett. 412: 388-396, 1997). The sequence of the segment is GTKVHMK (SEQ ID NO:31). This linker has the advantage that when it is present in native tetranectin, it binds the trimerization domain with the CRD domain, and therefore it is suitable for connecting the trimerization domain to another domain in general. Furthermore, the resulting construct is not expected to be more immunogenic than the construct without a linker.

Alternatively, a subsequence from the connecting strand 3 from human fibronectin can be chosen as a linker, corresponding to amino acids 1992-2102 (SWISSPROT numbering, entry P02751). The subsequence PGTSGQQPSVGQQ (SEQ ID NO:32) corresponding to amino acids number 2037-2049 is preferably used, and within that subsequence fragment GTSGQ (SEQ ID NO:33) corresponding to amino acids 2038-2042 is more preferable. This construct has the advantage that it not very prone to proteolytic cleavage and is not very immunogenic because fibronectin is present at high concentrations in plasma.

Alternatively, a suitable peptide linker can be based on the 10 amino acid residue sequence of the upper hinge region of murine IgG3. This peptide (PKPSTPPGSS, SEQ ID NO: 34) has been used to produce antibodies dimerized by means of a coiled helix (Pack P. and Pluckthun, A., 1992, Biochemistry 31:1579-1584) and can be useful as a spacer peptide according to the present invention. A corresponding sequence of the upper hinge region of human IgG3 can be even more preferable. Human IgG3 sequences are not expected to be immunogenic in human beings.

In a preferred embodiment, the linker peptide is selected from the group of the peptide of sequence APAETKAEPMT (SEQ ID NO:35) and of the peptide of sequence GAP.

Alternatively, the two components of the conjugates of the invention can be connected by a peptide the sequence of which contains a cleavage target for a protease, thus allowing the separation of component (i) from component (ii). Protease cleavage sites suitable for their incorporation into the polypeptides of the invention include enterokinase (cleavage site DDDDK, SEQ ID NO:36), factor Xa (cleavage site IEDGR, SEQ ID NO:37), thrombin (cleavage site LVPRGS, SEQ ID NO:38), TEV protease (cleavage site ENLYFQG, SEQ ID NO:39), PreScission protease (cleavage site LEVLFQGP, SEQ ID NO:40), inteins and the like. In a preferred embodiment, the cleavage site is a protease cleavage site expressed in tumor tissues, in inflamed tissues or in liver such that the separation of Apo A and of component (ii) takes place once the conjugate has reached the liver. In a preferred embodiment, the linker contains a matrix metalloprotease-9 recognition site (cleavage site LFPTS, SEQ ID NO:41).

The invention also contemplates conjugates wherein component (ii) comprises more than one antigen or epitope. In this case, the antigen or epitopes may be the same or different. In case the antigenic peptides are coupled to the PSM, then different peptides may be coupled at different sites of the PSM. Alternatively, the antigenic peptides may be forming a single polypeptide chain that may be coupled to a single site in the PSM

In a preferred embodiment, the conjugates of the invention may be formed by a single polypeptide chain wherein the C-terminus of component (ii) is a single antigenic peptide which forms a single polypeptide chain with component (i). In a still more preferred embodiment, the conjugate according of the invention is a single polypeptide chain wherein the C-terminus of component (ii) is coupled to the N-terminus of component (i) or wherein the N-terminus of component (ii) is coupled to the C-terminus of component (i).

In a preferred embodiment, the conjugates of the invention is not a peptide selected from the group of :
LMSCLILRIFILIKEGVISMAQDIISTIGDLVKWIIDTVNKFTKK (SEQ ID NO: 42)
and MAQDIISTIGDLVKWIIDTVNKFTKKKKKKKKKKKK (SEQ ID NO: 43)

### C. The cytotoxic effect of the conjugates of the invention

The conjugates of the invention are capable of inducing a cytolytic T cell (CTL) response having specificity for cells expressing on their surface peptide antigens that are presented in association with proteins encoded by the major histocompatibility complex (MHC). CTLs induce and promote the intracellular destruction of intracellular microbes, or the lysis of cells infected with such microbes or expressing tumoral antigens on the surface.

To determine whether a CTL response is obtained in an animal being treated in accordance with this invention, one measures the level of CD8+ cells (i.e. CTL) present in the blood or lymphatic organs such as the spleen or lymph nodes. This determination is done by first measuring the level of CD8+ cells before performing the method of this invention and measuring the level during treatment, e.g. at 7, 10, 20, 40 days, etc. The level or strength of the CD8+ (CTL) response can be assessed in vivo or in vitro.

In humans, there exists so far only one in vivo test to measure CD8+ T cell responses, which is a skin test. In this skin test, HLA class I binding peptides are injected intradermally. If a CTL response is present, these cells will recognize and attack peptide- pulsed dermal cells, causing a local inflammatory reaction either via cytokine release or the cytotoxic mechanism. This inflammatory reaction can be quantified by measuring the diameter of the local skin rash and/or by measuring the diameter of the infiltrate (i.e., the swelling reaction). As an alternative to the injection of soluble free peptide, the HLA-class I binding peptide can also be injected intradermally in a bound form, e.g., bound to extracorporally derived dendritic cells. In other mammals, additional, although experimental, in vivo tests to assess CD8+ T cell responses exist. For example, in a mouse model, CD8+ T cell responses can be measured by challenge infection with a vaccinia recombinant virus expressing the peptide used for immunization. The level of immunity to reinfection can be quantified as the factor of reduction of the vaccinia virus titer recovered from mouse organs after challenge infection

The level of CD8+ T cell responses can also be quantified in vitro, by estimating the number of CD8+ T cells specific for the antigenic peptide in question. In a naive mammal the so called "frequency", i.e., the number of specific CD8+ T cells divided by the number of non-specific white blood cells, is less than 10⁻⁶. After successful immunization, the frequency increases due to proliferation of specific T cells. During an acute viral infection, for example, the frequency of specific CD8+ T cells may rise to 10⁻² Then, after elimination of the virus, the frequency of specific CD8+ T cells usually drops to a "memory" level of around 10⁻⁴. Thus, the CD8+ T cell response can be quantified by measuring the frequency of specific CD8+ T cells. The higher the frequency, the stronger the response. The classical assays used to measure the frequency of specific CD8+ T cells are based on limiting dilution cell culture techniques, as described in detail by Kundig, T. M. et al. (Proc.Natl.Acad.Sci.USA., 1996, 93:9716-9723). A novel approach to estimate the frequency of specific CD8+ T cells is to construct soluble class I MHC (for use in mice) or HLA molecules (for use in humans) with a peptide bound to their groove, so that the specific T cell receptors will bind to these complexes. These complexes can be labeled for detection, for example, with a fluorescent substance, allowing for detection by flow cytometry. Another approach to quantify the level of CTL activity in vivo is the in vivo killing assay, which has been described in figures 3, 4 and 9 of the present patent invention.

### II. METHODS FOR OBTAINING THE CONJUGATES OF THE INVENTION

The conjugates of the invention can be obtained using any method known for a person skilled in the art. It is thus possible to obtain the PSM polypeptide or the variant of said protein by any standard method. For example, the PSM protein can be purified by phenol extraction of supernatants of stationary *S. epidermidis* as described in by Mehlin et al. (J.Exp.Med., 1999, 189:907). Alternatively, the PSM protein can be obtained from cDNA by means of expression in a heterologous organism such as, for example, *E.coli, S.cerevisiae, P.pastoris,* insect cells using methods known in the art such as those described in Otto, M. et al (J Infect Dis. 2004, 190:748-55).

Once there is a sufficient amount of purified PSM protein, it must be conjugated to the antigenic compound of interest. The conjugation of therapeutically active component (ii) to the PSM can be carried out in different ways. One possibility is the direct conjugation of a functional group to the therapeutically active component in a position which does not interfere with the activity of said component. As understood in the present invention, functional groups relate to a group of specific atoms in a molecule which are responsible for a characteristic chemical reaction of said molecule. Examples of functional groups include, but are not limited to hydroxy, aldehyde, alkyl, alkenyl, alkynyl, amide, carboxamide, primary, secondary, tertiary and quaternary amines, aminoxy, azide, azo (diimide), benzyl, carbonate, ester, ether, glyoxylyl, haloalkyl, haloformyl, imine, imide, ketone, maleimide, isocyanide, isocyanate, carbonyl, nitrate, nitrite, nitro, nitroso, peroxide, phenyl, phenyl, phosphine, phosphate, phosphono, pyridyl, sulfide, sulfonyl, sulfinyl, thioester, thiol and oxidized 3,4-dihydroxyphenylalanine (DOPA) groups. Examples of said groups are maleimide or glyoxylyl groups which react specifically with thiol groups in the PSM molecule and oxidized 3,4-dihydroxyphenylalanine (DOPA) groups which react with primary amine groups in the PSM molecule.

Another possibility is to conjugate therapeutically active component (ii) to the PSM molecule by means of the use of homo- or heterobifunctional groups. The bifunctional group can be conjugated first to the therapeutically active compound and, then, conjugated to the PSM peptide or, alternatively, it is possible to conjugate the bifunctional group to the PSM protein and then, conjugate it to the component (ii). Illustrative examples of theses types of conjugates include the conjugates known as ketone-oxime (described in US20050255042) in which the first component of the conjugate comprises an aminoxy group which is bound to a ketone group present in a heterobifunctional group which is in turn bound to an amino group in the second component of the conjugate.

In other embodiments, the agent which is used to conjugate components (i) and (ii) of the conjugates of the invention can be photolytically, chemically, thermally or enzymatically processed. It is particularly interesting to use linking agents which can be hydrolyzed by enzymes which are in the cell target, so that the therapeutically active compound is only released in the inside of the cell. Examples of types linking agents which can be intracellularly processed have been described in WO04054622, WO06107617, WO07046893 and W007112193.

In a preferred embodiment, component (ii) of the conjugate of the invention is a compound with a peptide nature, including both oligopeptides and peptides. Methods for chemically modifying a polypeptide chain are widely known for a person skilled in the art and include methods based on the conjugation through the thiol groups present in the cysteine moieties, methods based on the conjugation through the primary amino groups present in lysine moieties (US6809186), methods based on the conjugation through the N- and C-terminal moieties. Reagents suitable for modifying polypeptides to allow their coupling to other compounds include: glutaraldehyde (it allows binding compounds to the N-terminal end of polypeptides), carbodiimide (it allows binding the compound to the C-terminal end of a polypeptide), succinimide esters (for example MBS, SMCC) which allow activating the N-terminal end and cysteine moieties, benzidine (BDB), which allows activating tyrosine moieties, periodate, which allows activating carbohydrate moieties in the proteins which are glycosylated.

In the particular case in which component PSM and the antigenic peptide form a single peptide chain, it is possible to express the conjugate in a single step using a gene construct of the invention encoding said conjugate, for which said construct is introduced in a vector suitable for its expression in a heterologous organism together with transcription and, optionally, translation control elements. The transcription and, optionally, translation control elements present in the expression cassette of the invention include promoters, which direct the transcription of the nucleotide sequence to which they are operatively linked and other sequences which are necessary or suitable for the transcription and its suitable regulation in time and place, for example, initiation and termination signals, cleavage sites, polyadenylation signal, replication origin, transcriptional enhancers, transcriptional silencers, etc. Said elements, as well as the vectors used for constructing the expression cassettes and the recombinant vectors according to the invention are generally chosen according to the host cells to be used.

### III. POLYNUCLEOTIDES, GENE CONSTRUCTS, VECTORS AND HOST CELLS OF THE INVENTION.

In another aspect, the invention relates to a polynucleotide encoding a polypeptide of the invention. A person skilled in the art will understand that the polynucleotides of the invention will only encode the conjugates in which component (ii) has a peptide nature and which forms a single peptide chain with component (i), regardless of the relative orientation and regardless of the fact that both components are directly connected or separated by a spacer region.

In another aspect, the invention relates to a gene construct comprising a polynucleotide of the invention. The construct preferably comprises the polynucleotide of the invention located under the operative control of sequences regulating the expression of the polynucleotide of the invention. A person skilled in the art will understand that the polynucleotides of the invention must access the nucleus of a target tissue and there be transcribed and translated to give rise to the biologically active fusion protein.

In principle, any promoter can be used for the gene constructs of the present invention provided that said promoter is compatible with the cells in which the polynucleotide is to be expressed. Thus, promoters suitable for the embodiment of the present invention include, without being necessarily limited to, constitutive promoters such as the derivatives of the genomes of eukaryotic viruses such as the polyoma virus, adenovirus, SV40, CMV, avian sarcoma virus, hepatitis B virus, the promoter of the metallothionein gene, the promoter of the herpes simplex virus thymidine kinase gene, retrovirus LTR regions, the promoter of the immunoglobulin gene, the promoter of the actin gene, the promoter of the EF-lalpha gene as well as inducible promoters in which the expression of the protein depends on the addition of a molecule or an exogenous signal, such as the tetracycline system, the NFκB/UV light system, the Cre/Lox system and the promoter of heat shock genes, the regulatable promoters of RNA polymerase II described in WO/2006/135436 as well as tissue-specific promoters. In a preferred embodiment, the gene constructs of the invention contain the expression-enhancing regions present in promoter regions of predominantly hepatic expression genes such as human serum albumin genes, prothrombin genes, the alpha-1-microglobulin genes or aldolase genes, either in a single copy in the form of several copies thereof and either in an isolated form or in combination with other liver-specific expression elements such as cytomegalovirus, alpha-1-antitrypsin or albumin promoters. Preferably, the promoter used for expressing the polynucleotides of the invention are promoters functional in dendritic cells such as the fascin gene promoter as described by Bros et al. (J. Immunol., 2003, 171:1825-1834), the DC-CK1, DC-STAMP and DC-SIGN gene promoters, the Dectin-2 promoter described in Morita et al., (Gene Ther., 2001, 8:1729-37), the CD11c gene promoter as described in (Masood, R., et al. 2001. Int J Mol Med 8:335-343 and Somia, N. V., et al. 1995. Proc Acad Sci USA 92:7570-7574).

Other examples of promoters which are tissue-specific include the promoter of the albumin gene (Miyatake et al., 1997, J. Virol, 71:5124-32), the core promoter of hepatitis virus (Sandig et al, 1996, Gene Ther., 3:1002-9); the promoter of the alpha-phetoprotein gene (Arbuthnot et al., 1996, Hum.GeneTher., 7:1503-14), and the promoter of the globulin-binding protein which binds to thyroxine (Wang, L., et al., 1997, Proc. Natl. Acad. Sci. USA 94:11563-11566).

The polynucleotides of the invention or the gene constructs forming them can form part of a vector. Thus, in another aspect, the invention relates to a vector comprising a polynucleotide or a gene construct of the invention. A person skilled in the art will understand that there is no limitation as regards the type of vector which can be used because said vector can be a cloning vector suitable for propagation and for obtaining the polynucleotides or suitable gene constructs or expression vectors in different heterologous organisms suitable for purifying the conjugates. Thus, suitable vectors according to the present invention include expression vectors in prokaryotes such as pUC18, pUC19, Bluescript and their derivatives, mp18, mp19, pBR322, pMB9, CoIE1, pCR1, RP4, phages and shuttle vectors such as pSA3 and pAT28, expression vectors in yeasts such as vectors of the type of 2 micron plasmids, integration plasmids, YEP vectors, centromeric plasmids and the like, expression vectors in insect cells such as the pAC series and pVL series vectors, expression vectors in plants such as vectors of expression in plants such as pIBI, pEarleyGate, pAVA, pCAMBIA, pGSA, pGWB, pMDC, pMY, pORE series vectors and the like and expression vectors in superior eukaryotic cells based on viral vectors (adenoviruses, viruses associated to adenoviruses as well as retroviruses and lentiviruses) as well as non-viral vectors such as pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

The vector of the invention can be used to transform, transfect or infect cells which can be transformed, transfected or infected by said vector. Said cells can be prokaryotic or eukaryotic. By way of example, the vector wherein said DNA sequence is introduced can be a plasmid or a vector which, when it is introduced in a host cell, is integrated in the genome of said cell and replicates together with the chromosome (or chromosomes) in which it has been integrated. Said vector can be obtained by conventional methods known by the persons skilled in the art (Sambrok et al., 2001, mentioned above).

Therefore, in another aspect, the invention relates to a cell comprising a polynucleotide, a gene construct or a vector of the invention, for which said cell has been able to be transformed, transfected or infected with a construct or vector provided by this invention. The transformed, transfected or infected cells can be obtained by conventional methods known by persons skilled in the art (Sambrok et al., 2001, mentioned above). In a particular embodiment, said host cell is an animal cell transfected or infected with a suitable vector.

Host cells suitable for the expression of the conjugates of the invention include, without being limited to, mammal, plant, insect, fungal and bacterial cells. Bacterial cells include, without being limited to, Gram-positive bacterial cells such as species of the Bacillus, Streptomyces and Staphylococcus genus and Gram-negative bacterial cells such as cells of the Escherichia and Pseudomonas genus. Fungal cells preferably include cells of yeasts such as *Saccharomyces, Pichia pastoris* and *Hansenula polymorpha.* Insect cells include, without being limited to, Drosophila cells and Sf9 cells. Plant cells include, among others, cells of crop plants such as cereals, medicinal, ornamental or bulbous plants. Suitable mammal cells in the present invention include epithelial cell lines (porcine, etc.), osteosarcoma cell lines (human, etc.), neuroblastoma cell lines (human, etc.), epithelial carcinomas (human, etc.), glial cells (murine, etc.), hepatic cell lines (from monkey, etc.), CHO (Chinese Hamster Ovary) cells, COS cells, BHK cells, HeLa cells, 911, AT1080, A549, 293 or PER.C6, NTERA-2 human ECC cells, D3 cells of the mESC line, human embryonic stem cells such as HS293 and BGV01, SHEF1, SHEF2 and HS181, NIH3T3 cells, 293T, REH and MCF-7 and hMSC cells.

### IV. PHARMACEUTICAL PREPARATIONS OF THE INVENTION

The authors of the present invention have observed that that conjugates of the invention are capable of eliciting a CTL response towards the antigen forming part of the conjugate, thus allowing the use of said conjugates for the treatment of diseases wherein an immune response towards the antigen is required. Thus, in another aspect, the invention relates to a pharmaceutical preparation or a vaccine comprising a conjugate of the invention, a polynucleotide or gene construct of the invention, a vector of the invention, or a host cell of the invention and a pharmaceutically acceptable carrier.

The term "pharmaceutically acceptable carrier" refers to a carrier that does not cause an allergic reaction or other untoward effect in subjects to whom it is administered. Suitable pharmaceutically acceptable carriers include, for example, one or more of water, saline, phosphate buffered saline, dextrose, glycerol, ethanol, or the like and combinations thereof. In addition, if desired, the vaccine can contain minor amounts of auxiliary substances such as wetting or emulsifying agents, pH buffering agents, and/or adjuvants which enhance the effectiveness of the vaccine. Examples of adjuvants that may be effective include but are not limited to: aluminum hydroxide, N-acetyl-muramyl-L-threonyl-D-isoglutamine (thr-MDP), N- acetyl-nor-muramyl-L-alanyl-D-isoglutamine, MTP-PE and RIBI, which contains three components extracted from bacteria, monophosporyl lipid A, trehalose dimycolate and cell wall skeleton (MPL+TDM+CWS) in a 2% squalene/Tween 80 emulsion. Other examples of adjuvants include DDA (dimethyldioctadecylammonium bromide), Freund's complete and incomplete adjuvants and QuilA. In addition, immune modulating substances such as lymphokines (e.g., IFN-GAMMA, IL-2 and IL-12) or synthetic IFN-gamma inducers such as poly I:C can be used in combination with adjuvants described herein.

In yet another aspect, the invention relates to a conjugate of the invention, a polynucleotide or gene construct of the invention, a vector of the invention, a host cell or a pharmaceutically composition or a vaccine of the invention for use in medicine.

The conjugates may be used alone or may be delivered in combination with other antigens or with other compounds such as cytokines that are known to enhance immune stimulation of CTL responses, such as, GM-CSF, IL-12, IL-2, TNF, IFN, IL-18, IL-3, IL-4, IL-8, IL-9, IL-13, IL-10, IL-14, IL-15, G-SCF, IFN alpha, IFN beta, IFN gamma, TGFα, TNF, and the like. The cytokines are known in the art and are readily available in the literature or commercially.

In another aspect, the invention relates to a conjugate of the invention, to a polynucleotide or gene construct of the invention, to a vector of the invention, to a host cell of the invention or to a pharmaceutically composition or a vaccine according to the invention for use in a method of eliciting a cytotoxic response towards the antigenic peptide. Moreover, the invention relates to a method of eliciting an immune response in a subject in need of said response comprising the administration to said subject of a conjugate of the invention, to a polynucleotide or gene construct of the invention, to a vector of the invention, to a host cell of the invention or to a pharmaceutically composition or a vaccine according to the invention.

It will be appreciated that the conjugates, polynucleotides, gene constructs, vectors, host cells and pharmaceutical compositions of the invention can be used for the treatment of any disease for which an antigenic peptide has been identified that leads to the generation of an CTL-response. Thus, the compounds of the present invention are suitable for the treatment of viral diseases such as diseases resulting from infection by an adenovirus, a herpesvirus (e.g. HSV-1, HSV-II, CMV or VZV), a poxvirus (e.g., an orhopoxvirus such as variola or vaccinia, or molluscum contagiosum), a picornavirus (e.g. rhinvirus or enterovirus), an orthomyxovirus (e.g. influenza virus), a pramoyxovirus (e.g. parainfluenzavirus, mumps virus, measles virus and respiratory syncitial virus), a coronavirus (e.g. SARS), a papovirus (e.g. papillomavirus such as those causing genital warts, common warts or planter warts), a hepadnavisuts (e.g. hepatitis B virus), a flavivirus (e.g. hepatitis C virus or Dengue virus), or a retrovirus (e.g. lentivirus such as HIV-1).

In a preferred embodiment, the conjugates, polynucleotides, gene constructs, vectors, host cells or pharmaceutical preparations of the invention may be used for the treatment of infectious diseases caused by hepatitis C virus. These diseases include, but not limiting, cronic and acute hepatitis C.

Bacterial diseases such as diseases resulting from infection by bacteria of the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Heamophilus or Bordetella.

Other infectious diseases such as fungal diseases including but not limited to candidiasis, aspergillosis, histoplasmosis, cryptococcal meningitis, or parasitic diseases including but not limited to malaria, penumocystisn carinii, penumonia, leismaniosis, cryptoporidiosis, toxoplasmosis, and trypanosoma infection.

The conjugates of the invention are also suitable for the treatment of hyperproliferatuve diseases such as, without limitation, neoplasms located in the: colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvis, skin, soft tissue, spleen, thorax, and urogenital tract.

Similarly, other hyperproliferative disorders can also be treated or detected by the compounds of the invention include, but are not limited to acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-CeIl Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Normelanoma Skin Cancer, Non-Small Cell Lung Cancer, Occult Primary Metastatic Squamous Neck Cancer, Oropharyngeal Cancer, Osteo-ZMalignant Fibrous Sarcoma, OsteosarcomaWMalignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-CeIl Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

Moreover, the conjugates of the invention may also be suitable for the preventing and/or treating premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred.

Hyperplasia is a form of controlled cell proliferation, involving an increase in cell number in a tissue or organ, without significant alteration in structure or function. Hyperplastic disorders which can be prevented and/or treated with the compounds of the invention include, but are not limited to, angiofollicular mediastinal lymph node hyperplasia, angiolymphoid hyperplasia with eosinophilia, atypical melanocyte hyperplasia, basal cell hyperplasia, benign giant lymph node hyperplasia, cementum hyperplasia, congenital adrenal hyperplasia, congenital sebaceous hyperplasia, cystic hyperplasia, cystic hyperplasia of the breast, denture hyperplasia, ductal hyperplasia, endometrial hyperplasia, fibromuscular hyperplasia, focal epithelial hyperplasia, gingival hyperplasia, inflammatory fibrous hyperplasia, inflammatory papillary hyperplasia, intravascular papillary endothelial hyperplasia, nodular hyperplasia of prostate, nodular regenerative hyperplasia, pseudoepitheliomatous hyperplasia, senile sebaceous hyperplasia, and verrucous hyperplasia.

Metaplasia is a form of controlled cell growth in which one type of adult or fully differentiated cell substitutes for another type of adult cell. Metaplastic disorders which can be prevented and/or treated with the compounds of the invention include, but are not limited to, agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, and symptomatic myeloid metaplasia.

Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation. Dysplastic disorders which can be prevented and/or treated with the compounds of he invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, myoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be prevented and/or treated with the compounds according to the present invention include, but are not limited to, benign dysproliferative disorders (e.g., benign tumors, fibrocystic conditions, tissue hypertrophy, intestinal polyps, colon polyps, and esophageal dysplasia), leukoplakia, keratoses, Bowen's disease, Farmer's Skin, solar cheilitis, and solar keratosis.

In a preferred embodiment, the conjugates, polynucleotides, gene constructs, vectors, host cells and pharmaceutical preparations of the invention are used for the treatment of disorders caused by undesired cellular proliferation caused by human papillomavirus, including warts, as well as tumors. Wart types that can be treated with the compositions of the invention include common warts, plantar warts, genital warts, subungual or periungual warts and flat warts. Tumors that can be treated with the compositions of the present invention include cervical cancer, vulva, vagina and anus in woman and penis and anus in men.

### V. COMPOSITIONS OF THE INVENTION

The authors of the present invention have identified that the immunogenic effect of the conjugates of the invention is increased in a synergistic manner if the conjugates are administered together with other immunostimulatory compounds. Example 3 of the present application describes the ability of combinations of a PSM conjugates and either a TLR2 ligand (PGN), TLR3 ligand (pIC), a TLR4 ligand (LPS or the EDA domain of fibronectin), or a TLR9 ligand (CpG) to promote a cytotoxic response to the peptide coupled to the PSM which is stronger that the response obtained when the peptide is administered as a conjugate with PSM in the absence of additional TLR ligand.

Thus, in another aspect, the invention relates to a composition comprising, together or separately,
(a) a conjugate according to the invention, a polynucleotide or gene construct according to the invention, a vector according to the invention or a host cell according to the invention and
(b) a second component selected from the group of
   (i) one or more toll-like receptor agonists,
   (ii) one or more agonist antibodies of a co-stimulatory molecule,
   (iii) one of more cytokines and
   (iv) one or more compounds as defined in (i) to (iii)

The term "TLR agonist", as used herein, refers to a component which is capable of causing a signalling response through a TLR signalling pathway, either as a direct ligand or indirectly through generation of endogenous or exogenous ligand (Sabroe et al, JI 2003 p1630-5).

In one embodiment of the present invention, the TLR agonist is capable of causing a signalling response through TLR-1. Non-limiting examples of TLR-1 agonists include tri-acylated lipopeptides (LPs); phenol-soluble modulins; Mycobacterium tuberculosis LP; S-(2,3-bis(palmitoyloxy)-(2-RS)-propyl)-N-palmitoyl-(R)-Cys-(S)-Ser-(S)-Lys(4)-OH, trihydrochloride (Pam₃Cys) LP which mimics the acetylated amino terminus of a bacterial lipoprotein and OspA LP from *Borrelia burgdorfei.*

In an alternative embodiment, the TLR agonist is capable of causing a signalling response through TLR-2. Non-limiting examples of TLR-2 agonists include, without limitation, a phenol-soluble modulin (PSM) as defined in tables 1 and 2 and variants thereof, one or more of a bacterial lipopeptide from *M. tuberculosis, B. burgdorferi, T. pallidum;* peptidoglycans from species including *Staphylococcus aureus;* lipoteichoic acids, mannuronic acids, Neisseria porins, bacterial fimbriae, Yersina virulence factors, CMV virions, measles haemagglutinin, and zymosan from yeast.

In an alternative embodiment, the TLR agonist is capable of causing a signalling response through TLR-3, such as double stranded RNA, or polyinosinic-polycytidylic acid (Poly I:C).

In an alternative embodiment, the TLR agonist is capable of causing a signalling response through TLR-4, such as one or more of the EDA domain of fibronectin, a lipopolysaccharide (LPS) from gram-negative bacteria, or fragments thereof; heat shock protein (HSP) 10, 60, 65, 70, 75 or 90; surfactant Protein A, hyaluronan oligosaccharides, heparan sulphate fragments, fibronectin fragments, fibrinogen peptides and b-defensin-2. In one embodiment the TLR agonist is HSP 60, 70 or 90. In an alternative embodiment, the TLR agonist capable of causing a signalling response through TLR-4 is a non-toxic derivative of LPS such as monophosphoryl lipid A (MPL) as descrbed by Ribi et al (1986, Immunology and Immunopharmacology of bacterial endotoxins, Plenum Publ. Corp., NY, p407-419) and having the structure:

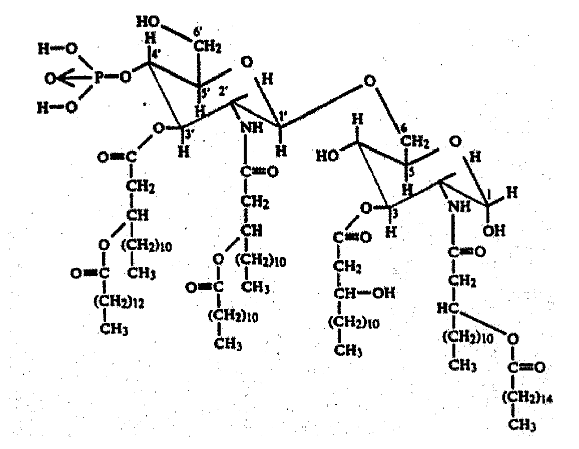

A further detoxified version of MPL results from the removal of the acyl chain from the 3- position of the disaccharide backbone, and is called 3-0-deacylated monophosphoryl lipid A (3D-MPL).

The non-toxic derivatives of LPS, or bacterial lipopolysaccharides, which may be used as TLR agonists in the present invention may be purified and processed from bacterial sources, or alternatively they may be synthetic. For example, purified monophosphoryl lipid A is described in Ribi et al 1986 (supra), and 3-0-Deacylated monophosphoryl or diphosphoryl lipid A derived from *Salmonella sp.* is described in GB 2220211 and US 4912094. Other purified and synthetic lipopolysaccharides have been described (US 6,005,099 and EP 0 729 473 B1; Hilgers et al., 1986, IntArch.Allergy.Immunol., 79(4):392-6; Hilgers et al., 1987, Immunology, 60(1):141-6; and EP 0 549 074 B1). Bacterial Iipopolysaccharide adjuvants may be 3D-MPL and the f3(1-6) glucosamine disaccharides described in US 6,005,099 and EP 0 729 473 B1. Accordingly, other LPS derivatives that may be used as TLR agonists in the present invention are those immunostimulants that are similar in structure to that of LPS or MPL or 3D-MPL. In another aspect of the present invention the LPS derivatives may be an acylated monosaccharide, which is a sub-portion to the above structure of MPL. A disaccharide agonist may be a purified or synthetic lipid A of the following formula: 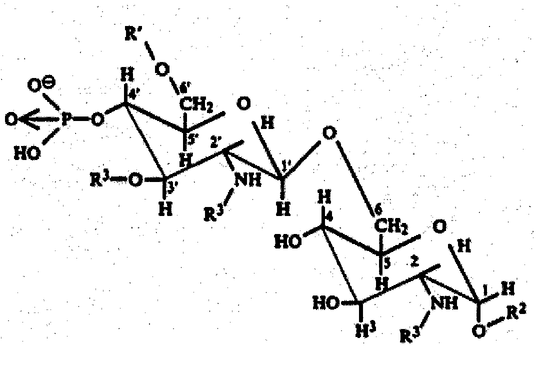 wherein R² may be H or PO₃H₂; R³ may be an acyl chain or 8-hydroxymyristoyl or a 3-acyloxyacyl residue having the formula: 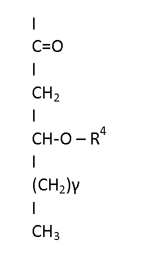 wherein R⁴ is 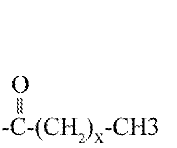 and X and Y have a value of 0 up to 20.

A yet further non-toxic derivative of LPS, which shares little structural homology with LPS and is purely synthetic is that described in WO 00/00462, the contents of which are fully incorporated herein by reference.

In an alternative embodiment, the TLR agonist is capable of causing a signalling response through TLR-5, such as bacterial flagellin.

In an alternative embodiment, the TLR agonist is capable of causing a signalling response through TLR-6 such as mycobacterial lipoprotein, di-acylated LP, and phenol-soluble modulin. Further TLR6 agonists are described in W02003043572.

In an alternative embodiment, the TLR agonist is capable of causing a signalling response through TLR-7 such as loxoribine, a guanosine analogue at positions N7 and C8, or an imidazoquinoline compound, or derivative thereof. In one embodiment, the TLR agonist is imiquimod. Further TLR7 agonists are described in W002085905.

In an alternative embodiment, the TLR agonist is capable of causing a signalling response through TLR-8 such as an imidazoquinoline molecule with anti-viral activity, for example resiquimod (R848); resiquimod is also capable of recognition by TLR-7. Other TLR-8 agonists which may be used include those described in W02004071459.

In an alternative embodiment, the TLR agonist is capable of causing a signalling response through TLR-9 such as s DNA containing unmethylated CpG nucleotides, in particular sequence contexts known as CpG motifs. CpG-containing oligonucleotides induce a predominantly Th1 response. Such oligonucleotides are well known and are described, for example, in WO 96/02555, WO 99/33488 and U.S. Patent Nos. 6,008,200 and 5,856,462. In one embodiment, CpG nucleotides are CpG oligonucleotides.

In an alternative embodiment, component (i) is a TLR agonist capable of causing a signalling response through TLR-10. Alternatively, the TLR agonist is capable of causing a signalling response through any combination of two or more of the above TLRs.

The term "agonistic antibodies" of a co-stimulatory molecule", as used herein, relates to compounds which are capable of binding with high affinity to proteins in the surface of the T-cells and which have a agonistic effect, i.e. they are capable of activating signaling through the receptor leading to the same effects as the binding of the ligand. Preferred co-stimulatory molecules that can be targeted with the agonistic antibodies are molecules having a co-stimultory activity on T-cell proliferation and include, without limitation, CD4 and CD137. Thus, preferred agonistic antibodies suitable for use in the compositions of the invention include anti-CD4 and anti-CD137-specific antibodies. According to the present invention, the term, antibody" comprises complete antibody molecules as well as fragments thereof being capable of binding to CD137 or CD4, and thus exerting an agonistic effect on CD137 or CD4 function. Activation of CD137 co-stimulates proliferation of T lymphocytes (Goodwin et al., 1993; Pollock et al., 1993; Schwarz et al., 1996), and CD137 ligand expressed by B lymphocytes co-stimulates T cell proliferation synergistically with B7 (DeBenedette et al., 1995).

Antibodies for use in the present invention may be directed against CD137 or CD4. The term "antibody" comprises polyclonal as well as monoclonal antibodies, chimeric antibodies, humanised antibodies, which may be present in bound or soluble form. Furthermore, an "antibody" according to the present invention may be a fragment or derivative of the afore-mentioned species. Such antibodies or antibody fragments may also be present as recombinant molecules, e. g. as fusion proteins with other (proteinaceous) components. Antibody fragments are typically produced through enzymatic digestion, protein synthesis or by recombinant technologies known to a person skilled in the art. Therefore, antibodies for use in the present invention may be polyclonal, monoclonal, human or humanised or recombinant antibodies or fragments thereof as well as single chain antibodies, e. g. scFv- constructs, or synthetic antibodies.

Polyclonal antibodies are heterogenous mixtures of antibody molecules being produced from sera of animals which have been immunised with the antigen. Subject of the present invention are also polyclonal monospecific antibodies which are obtained by purification of the antibody mixture (e.g. via chromatography over a column carrying peptides of the specific epitope. A monoclonal antibody represents a homogenous population of antibodies specific for a single epitope of the antigen.

Monoclonal antibodies can be prepared according to methods described in the prior art (e. g. Kohler und Milstein, Nature, 256,495-397, (1975) ; US-Patent 4,376, 110; Harlow und Lane, Antibodies: A Laboratory Manual, Cold Spring, Harbor Laboratory (1988); Ausubel et al., (eds), 1998, Current Protocols in Molecular Biology, John Wiley amp; Sons, New York).

Genetically engineered antibodies for use in the present invention may be produced according to methods as described in the afore-mentioned references.

Antibodies for use in the present invention can belong to any one of the following classes of immunoglobulins : IgG, IgM, IgE, IgA, GILD and, where applicable, a subclass of the afore-mentioned classes, e. g. the sub-classes of the IgG class. IgG and ist sub-classes, such as IgG1, IgG2, IgG2a, IgG2b, IgG3 or IgGM, are preferred. IgG subtypes IgG1/k or IgG2b/k are especially preferred. A hybridoma clone which produces monoclonal antibodies for use in the present invention can be cultured in vitro, in situ oder in vivo. High titers of monoclonal antibodies are preferably produced in vivo or in situ.

Chimeric antibodies are species containing components of different origin (e. g. antibodies containing a variable region derived from a murine monoclonal antibody, and a constant region derived from a human immunoglobulin). Chimeric antibodies are employed in order to reduce the immunogenicity of the species when administered to the patient and to improve the production yield. For example, in comparison to hybridoma cell lines, murine monoclonal antibodies give higher yiels. However, they lead to a higher immunogenicity in a human patient. Therefore, chimeric human/murine antibodies are preferably used. Even more preferred is a monoclonal antibody in which the hypervariable complementarity defining regions (CDR) of a murine monoclonal antibody are combined with the further antibody regions of a human antibody. Such an antibody is called a humanised antibody. Chimeric antibodies and methods for their production are described in the prior art (Cabilly et al., Proc. Natl. Sci. USA 81: 3273-3277 (1984); Morrison et al. Proc. Natl. Acad. Sci USA 81: 6851-6855 (1984); Boulianne et al. Nature 312 643-646 (1984); Cabilly et al., EP-A-125023; Neuberger et al., Nature 314: 268-270 (1985); Taniguchi et al., EP-A-171496; Morrion et al., EP-A-173494; Neuberger et al., WO 86/01533; Kudo et al., EP-A-184187; Sahagan et al., J. Immunol. 137: 1066-1074 (1986); Robinson et al., WO 87/02671; Liu et al., Proc. Natl. Acad. Sci USA 84: 3439-3443 (1987); Sun et al., Proc. Natl. Acad. Sci USA 84: 214218 (1987); Better et al., Science 240: 1041-1043 (1988) und Harlow und Lane, Antibodies: A Laboratory Manual, supra).

Antibody fragments comprise any deleted or derivatised antibody moieties having one or two binding site (s) for the antigen, i. e. one or more epitopes of CD137 or CD137 ligand.

Specific examples of such antibody framents are Fv, Fab or F (ab') 2 fragments or single strand fragments such as scFv. Double stranded fragments such as Fv, Fab or F (ab') 2 are preferred. Fab und F (ab') 2 fragments have no Fc fragment contained in intact antibodies. As a beneficial consequence, such fragments are transported faster in the circulatory system and show less non-specific tissue binding in comparison to complete antibody species. Such fragments may be produced from intact antibodies by proteolytic digestion using proteases such as papain (for the production of Fab fragments) or pepsin (for the production of F (ab') 2 fragments), or chemical oxidation.

Preferably, antibody fragments or antibody constructs are produced through genetic manipulation of the corresponding antibody genes. Recombinant antibody constructs usually comprise single-chain Fv molecules (scFvs,-30kDa in size), in which the VH and VL domains are tethered together via a polypeptide linker to improve expression and folding efficiency. In order to increase functional affinity (avidity) and to increase the size and thereby reduce the blood clearance rates, the monomeric scFv fragments can be complexed into dimers, trimers or larger aggregates using adhesive protein domains or peptide linkers. An example of such a construct of a bivalent scFv dimer is a 60 kDa diabody in which a short, e. g. five-residue, linker between VH~ and V-domains of each scFv prevents alignment of V-domains into a single Fv module and instead results in association of two scFv molecules. Diabodies have two functional antigen-binding sites. The linkers can also be reduced to less than three residues which prevent the formation of a diabody and instead directs three scFv molecules to associate into a trimer (90 kDa triabody) with three functional antigen-binding sites. Association of four scFvs into a tetravalent tetrabody is also possible. Further preferred antibody constructs for use in the present invention are dimers of scFv-CH3 fusion proteins (80 kDa; so-called"minibodies") Antibodies for use in the present invention are preferably directed to a peptide or protein which is encoded by a nucleic acid comprising a nucleotide sequence according to GenBank Acc. No. L12964 8 (see Fig. 8A) or a nucleic acid having at least 90%, preferably at least 95%, especially preferred at least 97% homology to the nucleotide sequence according to GenBank Acc. No. L12964.

The term "immunostimulatory cytokine", as used herein, is understood as any compound which promotes an increase in the activity of any component of the immune system including those components forming part or being involved in cell-mediated immune response, humoral-mediated immune response and the complement system. Preferably, the immunostimulatory cytokine is selected from the group of IL-12, IL-2, IL-15, IL-18, IL-24, GM-CSF, TNFα, CD40 ligand, IFNα, IFNβ, IFNγ and functionally equivalent variants thereof.

In a preferred embodiment, component (b) of the compositions of the invention includes a TLR agonist. In a still more preferred embodiment, the agonist is selected from the group of a TLR3 ligand, a TLR4 ligand and a TLR9 ligand. In a still more preferred embodiment, the TLR3 ligand is polyI:C, the TLR4 ligand is LPS or the EDA domain of fibronectin and/or the TLR9 ligand is CpG.

### VI. MEDICAL USES OF THE COMPOSITIONS OF THE INVENTION

The compositions of the invention are suitable for promoting an immunological response towards the antigenic peptide forming part of the conjugates. Thus, in another aspect, the invention relates to pharmaceutical preparation or a vaccine comprising a composition of the invention and a pharmaceutically acceptable carrier. In another aspect, the invention relates to a composition of the invention or a pharmaceutical preparation or vaccine comprising the compositions of the invention for use in medicine.

The compositions of the invention may be used for the same purposes than the conjugates of the invention. Thus, in another aspect, the invention relates to the composition of the invention for use in medicine.

The compositions of the invention may be used in vivo as a vaccine for inducing a cytotoxic response towards the antigenic peptide. Thus, in another aspect, the invention relates to a method for inducing a cytotoxic response towards an antigenic peptide comprising administering to a subject in need of said response the compositions of the invention. In another aspect, the invention relates to the compositions and pharmaceutical compositions of the invention for use in a method of inducing a cytotoxic response towards an antigenic peptide. In a preferred embodiment, the invention relates to a method to induce a cytotoxic response against an infectious, allergic or neoplastic disease.

Thus, in another aspect, the invention relates to a composition of the invention for use in the treatment of an infectious disease, an allergic disease or a neoplastic disease. In another aspect, the invention relates to a method for the treatment of an infectious disease, an allergic disease or a neoplastic disease which comprises the administration to a patient in need thereof the compositions of the invention

### VII. IN VITRO USES OF THE CONJUGATES AND COMPOSITIONS OF THE INVENTION

As shown in example 2 of the present invention, the conjugates of the invention are capable of promoting maturation of dendritic cells and antigenic presentation. Thus, the conjugates of the invention may also be used in vitro for promoting maturation of dendritic cells. Thus, in another aspect, the invention relates to the use of a conjugate as of the invention, a polynucleotide or gene construct of the invention, a vector of the invention, a host cell of the invention or a composition of the invention for promoting the presentation of the antigenic peptide or peptides by antigen presenting cells or for promoting maturation of antigen-presenting cells. In a preferred embodiment, the antigen-presenting cells are dendritic cells.

The different embodiments relating to the methods for promoting maturation of dendritic cells and antigen presentation are summarized in detail below under item VIII.

### VIII. METHOD FOR THE PREPARATION OF ANTIGEN-PRIMED ANTIGEN PRESENTING CELLS AND ANTIGEN PRESENTING CELLS OBTAINED USING SAID METHOD

The authors of the present invention have also observed that the conjugates of the invention are capable of promoting maturation of dendritic cells and the antigenic presentation by said cells, thus leading to antigen-primed dendritic cells. Thus, in another aspect, the invention relates to a method for obtaining antigen-primed dendritic cells comprising the steps of
(i) contacting an antigen-presenting (APC) cell with a conjugate, a polynucleotide, a gene construct, a vector, a host cell or a pharmaceutical composition or a preparation according to the invention and
(ii) isolating the antigen-loaded APC.

In the first step, the method of obtaining antigen-primed APC involves contacting the APC with a conjugate according to the invention.

The term "antigen presenting cells (APCs), as used herein, refers to a class of cells capable of presenting one or more antigens in the form of peptide-MHC complex recognizable by specific effector cells of the immune system, and thereby inducing an effective cellular immune response against the antigen or antigens being presented. APC suitable for use in the present invention include both professional APC such as dendritic cells (DC), macrophages and B-cells as well as non-professional APC such as fibroblasts, thymic epithelial cells, thyroid epithelial cells, glial cells, pancreatic beta cells and vascular endothelial cells. In a preferred embodiment, the APC are dendritic cells. Preferably, the APC for use in the methods of the inventions are dendritic cells, since these are the only APC having the capacity to present antigens in an efficient amount to activate naive T-cells for cytotoxic T-lymphocyte (CTL) responses.

The term "dendritic cells (DCs)" refers to a diverse population of morphologically similar cell types found in a variety of lymphoid and non-lymphoid tissues, Steinman (1991) Ann. Rev. Immunol. 9:271-296. Dendritic cells constitute the most potent and preferred APCs in the organism. While the dendritic cells can be differentiated from monocytes, they possess distinct phenotypes. For example, a particular differentiating marker, CD14 antigen, is not found in dendritic cells but is possessed by monocytes. Also, mature dendritic cells are not phagocytic, whereas the monocytes are strongly phagocytosing cells. It has been shown that mature DCs can provide all the signals necessary for T cell activation and proliferation.

Dendritic cells can be isolated or generated from blood or bone marrow, or secondary lymphoid organs of the subject, such as but not limited to spleen, lymph nodes, tonsils, Peyer's patch of the intestine, and bone marrow, by any of the methods known in the art. Preferably, DCs used in the methods of the invention are (or terminally differentiated) dendritic cells. The source of dendritic cells is preferably human blood monocytes.

Immune cells obtained from such sources typically comprise predominantly recirculating lymphocytes and macrophages at various stages of differentiation and maturation. Dendritic cell preparations can be enriched by standard techniques (see e.g., Current Protocols in Immunology, 7.32.1-7.32.16, John Wiley and Sons, Inc., 1997) such as by depletion of T cells and adherent cells, followed by density gradient centrifugation. DCs may optionally be further purified by sorting of fluorescence-labeled cells, or by using anti-CD83 MAb magnetic beads. Alternatively, a high yield of a relatively homogenous population of DCs can be obtained by treating DC progenitors present in blood samples or bone marrow with cytokines, such as granulocyte-macrophage colony stimulating factor (GM-CSF) and interleukin 4 (IL-4). Under such conditions, monocytes differentiate into dendritic cells without cell proliferation. Further treatment with agents such as TNF alpha stimulates terminal differentiation of DCs.

By way of example but not limitation, dendritic cells can be obtained from blood monocytes as follows: peripheral blood monocytes are obtained by standard methods (see, e.g., Sallusto et al., 1994, J. Exp. Med. 179:1109-1118). Leukocytes from healthy blood donors are collected by leukapheresis pack or buffy coat preparation using Ficoll-Paque density gradient centrifugation and plastic adherence. If mature DCs are desired, the following protocol may be used to culture DCs. Cells are allowed to adhere to plastic dishes for 4 hours at 37 degrees C. Nonadherent cells are removed and adherent monocytes are cultured for 7 days in culture media containing 0.1 mu g/ml granulocyte-monocyte colony stimulating factor (GM-CSF) and 0.05 mu g/ml interleukin-4 (IL-4). In order to prepare mature dendritic cells, tumor necrosis factor- alpha is added on day 5, and cells are collected on day 7.

Methods for maturing immature dendritic cells are known in the art. For example, fully and irreversibly mature and stable DCs can be obtained by culturing immature dendritic cells in medium with autologous or non-autologous monocyte-conditioned medium, PBMC-conditioned medium or SACs as described in Romani et al. (Immunol. Methods, 1996, 196:137) and Bender et al. (J. Immunol. Methods, 1996, 196:121). Jonuleit et al. (Eur. J. Immunol., 1997 12:3135-3142) disclose maturation of immature DCs by overnight culture in medium containing GM-CSF and IL-4, plus a "cytokine cocktail" comprising TNF-I±, IL-1 I², IL-6 and PGE2. Preferred concentrations of cytokines in the cocktail are 10 ng/ml TNF-I±, 10 ng/ml IL- 11², 100 ng/ml IL-6 and 1 I¼g/ml PGE2. European Patent Publication EP-A-O 922 758 discloses the production of mature dendritic cells from immature dendritic cells derived from monocytes by culture in medium containing IFN-I. U.S. Patent Publication No. 2004/0152191 discloses the maturation of dendritic cells by culture with RU 41740. The "CD40L base process" and the "Post-Maturation Electroporation (PME) CD40L process" for DC maturation are described in International Application WO 2006/042177, the contents of which are incorporated by reference. In the CD40L base process, immature DC are transfected with CD40L mRNA and antigen-encoding mRNA, and then treated with IFN-I (1000 U/ml) or TNF-I± (10 ng/ml) or a combination of IFN-I³ and PGE2 (1 I¼g/ml). The cytokine levels may be increased or decreased. In the "PME-CD40L process", monocyte derived immature DCs are matured (typically after 4-7 days after beginning culture of monocytes with GM-CSF and IL-4) by overnight culture (12-30 hours, preferably about 18 hrs) with TNF-I± (10 ng/ml), IFN-I³ (1000 U/ml) and PGE2 (1 I¼g/ml). Following this overnight culture, DCs are harvested and electroporated with antigen-encoding RNA and CD40L mRNA and cultured in X-VIVO 15 media containing 800 U/ml GM-CSF and 500 U/ml IL-4 for 4 hrs or more hours prior to removing and aliquot for pulsing with a lysate or extract of cells or virions.

Alternatively, the antigen-presenting cells (APCs), including but not limited to macrophages, dendritic cells and B-cells, can be obtained by production *in vitro* from stem and progenitor cells from human peripheral blood or bone marrow as described by Inaba et al, (1992) J Exp Med 176 1693-1702.

Dendritic cells obtained in this way characteristically express the cell surface marker CD83. In addition, such cells characteristically express high levels of MHC class II molecules, as well as cell surface markers CD1 alpha, CD40, CD86, CD54, and CD80, but lose expression of CD14. Other cell surface markers characteristically include the T cell markers CD2 and CD5, the B cell marker CD7 and the myeloid cell markers CD13, CD32 (Fc gamma R II), CD33, CD36, and CD63, as well as a large number of leukocyte-associated antigens.

Optionally, standard techniques, such as morphological observation and immunochemical staining, can be used to verify the presence of dendritic cells. For example, the purity of dendritic cells can be assessed by flow cytometry using fluorochrome-labeled antibodies directed against one or more of the characteristic cell surface markers noted above, e.g., CD83, HLA-ABC, HLA-DR, CD1 alpha, CD40, and/or CD54. This technique can also be used to distinguish between immature and mature DCs, using fluorochrome-labeled antibodies directed against CD 14, which is present in immature, but not mature, DCs.

DC precursors may be obtained from a healthy subject or a subject known to be suffering from a disease associated with the expression of a particular antigen. Such DC precursors may be allogeneic or autologous.

Once DC precursors are obtained, they are cultured under appropriate conditions and for a time sufficient to expand the cell population and maintain the DC's in a state for optimal antigen uptake, processing and presentation. In one preferred approach to culture of DC precursors, DC are generated from such DC precursors by culture ex vivo in serum free or protein-free medium for 40 hours, in the absence of exogenously added cytokines, as detailed in co-owned USSN 60/158, 618.

Preferred aspects of DC isolation and culture include the use of culture medium lacking exogenously supplied cytokines and culture under serum-free conditions in a manner effective to result in the generation of Ag-loaded superactivated DC, which are cells that have already processed an Ag and have the ability to present the Ag to the immune cells and quickly generate Ag-specific immune responses, e. g., CTL-mediated T cell responses to tumor antigens.

Dendritic cells can be preserved by cryopreservation either before or after exposure to the conjugates of the invention.

In step (i) of the method of obtaining antigen-loaded dendritic cells of the invention, the APC are contacted contacting a dendritic cell with a conjugate, a polynucleotide, a gene construct, a vector, a host cell or a pharmaceutical composition according to the invention. The contacting step will be carried out differently depending on whether the DC are contacted with the conjugates as such or with a nucleic acid encoding said conjugates.

In the case that the contacting step is carried out using the conjugates of the invention, the contacting step comprises the contacting/incubating of the DC with the conjugates for sufficient time. In one embodiment, sensitization may be increased by contacting the APCs with heat shock protein(s) (hsp) noncovalently bound to the conjugate. It has been demonstrated that hsps noncovalently bound to antigenic molecules can increase APC sensitization.

Alternatively, the APC can be transfected with a vector encoding the conjugates and used for adoptive immunotherapy and/or vaccine therapy. Several approaches for introducing nucleic acids into cells in vivo, ex vivo and in vitro have been used such as lipid or liposome based gene delivery and replication-defective retroviral vectors harboring a therapeutic polynucleotide sequence as part of the retroviral genome. Widely used retroviral vectors include those based upon murine leukemia virus (MuLV), gibbon ape leukemia virus (GaLV), Simian Immunodeficiency virus (SIV), human immunodeficiency virus (HIV), alphavirus, and combinations thereof The vectors are optionally pseudotyped to extend the host range of the vector to cells which are not infected by the retrovirus corresponding to the vector. For example, the vesicular stomatitis virus envelope glycoprotein (VSV-G) has been used to construct VSV-G-pseudotyped HIV vectors which can infect hematopoietic stem cells. Adeno-associated virus (AAV)-based vectors are also used to transduce cells with target nucleic acids, e.g., in the in vitro production of nucleic acids and peptides, and in *in vivo* and *ex vivo* gene therapy procedures. Other suitable viral vectors include herpes virus, lentivirus, and vaccinia virus.

In addition to viral vectors, a number of non-viral transfection methods are available. Such methods include, but are not limited to electroporation methods, calcium phosphate transfection, liposomes, cationic lipid complexes, water-oil emulsions, polethylene imines, and dendrimers. Where appropriate, two or more types of vectors can be used together. For example, a plasmid vector may be used in conjunction with liposomes. In the case of non- viral vectors, nucleic acid may be incorporated into the non-viral vectors by any suitable means known in the art. For plasmids, this typically involves ligating the construct into a suitable restriction site. For vectors such as liposomes, water-oil emulsions, polyethylene amines and dendrimers, the vector and construct may be associated by mixing under suitable conditions known in the art.

It is also possible to load APC with RNA. This is usually carried out using techniques such as electroporation, passive uptake, lipofection, microinjection, cationic reagents, viral transduction, CaPO4 and the like

The activation of the DC can be detected by contacting the DC with T cell clones expressing a T-cell receptor specific for the antigenic peptide present in the conjugate and measuring the proliferation of the T-cells, usually by measuring the incorporation of a labeled nucleotide analog.

Once the APC have been sensitized with the antigen, the cells are isolated in order to obtain the antigen-primed APC. Cell surface markers can be used to isolate the cells necessary to practice the methods of this invention. For example, DCs express MHC molecules and costimulatory molecules (e.g., B7-1 and B7-2). The expression of surface markers facilitates identification and purification of these cells. These methods of identification and isolation include FACS, column chromatography and the like. For a review of immunological and immunoassay procedures in general, see Stites and Terr (eds.) 1991 Basic and Clinical Immunology (7th ed.) and Paul supra. For a discussion of how to make antibodies to selected antigens see Harlow and Lane (1989) supra. Cell isolation or immunoassays for detection of cells during cell purification can be performed in any of several configurations, e.g., those reviewed in Maggio (ed.) (1980) Enzyme Immunoassay, CRC Press, Boca Raton, FIa.; Tijan (1985) "Practice and Theory of Enzyme Immunoassays," Laboratory Techniques in Biochemistry and Molecular Biology, Elsevier Science Publishers B.V., Amsterdam; Harlow and Lane, supra; Chan (ed.) (1987) Immunoassay: A Practical Guide Academic Press, Orlando, FIa.; Price and Newman (eds.) (1991) Principles and Practice of Immunoassays, Stockton Press, NY; and Ngo (ed.) (1988) Non-isotopic Immunoassays, Plenum Press, NY. Cells can be isolated and characterized by flow cytometry methods and FACS analysis. A wide variety of flow-cytometry methods are known. For a general overview of fluorescence activated flow cytometry, see, for example, Abbas et al. (1991) Cellular and Molecular immunology W.B. Saunders Company, particularly chapter 3, and Kuby (1992) Immunology W. H. Freeman and Company, particularly chapter 6.

Labeling agents which can be used to label cell antigen include, but are not limited to monoclonal antibodies, polyclonal antibodies, proteins, or other polymers such as affinity matrices, carbohydrates or lipids. Detection proceeds by any known method, such as immunoblotting, western blot analysis, tracking of radioactive or bioluminescent markers, capillary electrophoresis, or other methods which track a molecule based upon size, charge or affinity.

Thus, in another aspect, the invention relates to an APC obtainable by the method previously mentioned.

The antigen-loaded APC obtained using the method of the present invention can be used to activate CD8+ T cells and/or CD4+ T-cells in vitro or can be introduced directly in a subject to activate the T cells in vivo. Thus, in further aspects, the invention relates to an APC obtainable by the method of the invention for use in medicine as well as to a vaccine comprising the APC obtainable by the method of the invention. It will be understood that, for the purposes of medical uses, the cells may originate from the same individual which is to be trated (autologous transplantation) or from a different individual (allogeneic transplantation). In allogeneic transplantation, the donor and recipient are matched based on similarity of HLA antigens in order to minimize the immune response of both donor and recipient cells against the other.

CD8⁺ T cells educated in vitro can be introduced into a mammal where they are cytotoxic against target cells bearing antigenic peptides corresponding to those the T cells are activated to recognize on class I MHC molecules. These target cells are typically cancer cells, or pathogen-infected cells which express unique antigenic peptides on their MHC class I surfaces.

Similarly, CD4⁺ helper T cells, which recognize antigenic peptides in the context of MHC class II, can also be stimulated by the APCs of the invention, which comprise antigenic peptides both in the context of class I and class II MHC. Helper T cells also stimulate an immune response against a target cell. As with cytotoxic T cells, helper T cells are stimulated with the antigen-loaded APCs *in vitro* or *in vivo*.

Thus, in another aspect, the invention relates to an antigen-presenting cell according to the invention, to use in a method for induction of a cytotoxic cell response against the cytotoxic antigen. In another aspect, the invention relates to an antigen-presenting cell according to the invention to use in a method for induction of a cytotoxic cell response against an infectious, allergic or neoplastic disease.

The immunogenicity of the antigen-presenting cells or educated T cells produced by the methods of the invention can be determined by well known methodologies including, but not limited to the following:
- ⁵¹Cr-release lysis assay for CTL function. Cytotoxic T cells can kill cells that present the particular peptide:MHC class I complex that they specifically recognize. CTL function is typically determined by measuring the release of radioactive isotope by a target cell (e.g., an antigen loaded APC, tumor cell, pathogen cell, etc.).
- Cytokine-release assay. Analysis of the types and quantities of cytokines secreted by T cells upon contacting modified APCs can be a measure of functional activity. Cytokines can be measured by ELISA or ELISpot assays to determine the rate and total amount of cytokine production (Fujihashi et a/. (1993) J. Immunol. Meth. 160:181; Tanquay and Killion (1 994) Lymphokine Cytokine Res. 13:259).
- In *vitro* T-cell education. The compositions of the invention can be assayed for the ability to elicit reactive T-cell populations from normal donor or patient-derived PBMC. In this system, elicited T cells can be tested for lytic activity, cytokine-release, polyclonality, and crossreactivity to the antigenic epitope (Parkhurst et al., 1996, J. Immunol. 1996, 157:2539).
- Transgenic animal models. Immunogenicity can be assessed *in vivo* by vaccinating HLA transgenic mice with the compositions of the invention and determining the nature and magnitude of the induced immune response. Alternatively, the hu-PBL-SCID mouse model allows reconstitution of a human immune system in a mouse by adoptive transfer of human PBL. These animals may be vaccinated with the compositions and analyzed for immune response as previously mentioned in Shirai et al. (1995) J. Immunol. 154:2733; Mosier et al.(1993) Proc. Natl. Acad. Sci. USA 90:2443.
- Proliferation Assays. T cells will proliferate in response to reactive compositions. Proliferation can be monitored quantitatively by measuring, for example, [³H]-thymidine-uptake. In a preferred method, T-cell proliferation is measured using carboxy-flouroscein diacetate succinimidyl ester (CFSE). CFSE consists of a fluorescein molecule containing a succinimidyl ester functional group and two acetate moieties. Lymphocytes are first incubated with membrane permeable, non-fluorescent CFSE which passively diffuses into cells and intracellular esterases cleave the acetate groups converting it to a fluorescent, membrane impermeant dye. Excess dye is washed away and quiescent cells are induced to proliferate by *in vitro* mitogenic or antigenic stimulation. The cells are maintained in culture for six days. During each round of cell division, the CFSE fluorescence is halved, allowing the identification of successive cell generations. CFSE is detected using standard fluorescein filters (excitation = 492 nm, emission = 517 nm). Staining with fluorescence labelled antibodies for cell surface molecules, such as CD4 and CD8, and intracellular markers allows the examination of the proliferation of specific lymphocyte subsets as well as the characterization of the phenotypic and functional properties of proliferating cells using flow cytometry. The concomitant use of propidium iodide (PI) facilitates the assessment of cell viability. CFSE flow kits are available through Renovar, Inc. (Madison, WI) and other sources.
- Primate models. A non-human primate (chimpanzee) model system can be used to monitor *in vivo* immunogenicities of HLA-restricted ligands. It has been demonstrated that chimpanzees share overlapping MHC-ligand specificities with human MHC molecules, thus allowing one to test HLA-restricted ligands for relative *in vivo* immunogenicity (Bertoni et al., 1998, Immunol. 161 :4447).
- Monitoring TCR Siqnal Transduction Events. Several intracellular signal transduction events (e.g., phosphorylation) are associated with successful TCR engagement by MHC-ligand complexes. The qualitative and quantitative analyses of these events have been correlated with the relative abilities of compositions to activate effector cells through TCR engagement (Salazar et al. (2000) Tnt. J. Cancer 85829; lsakov et al. (1995) J. Exp. Med. 181 :375).

The APC cells can be used for the treatment of different diseases depending on the type of antigen which form part of the conjugates used for the sensitization of the antigen-presenting cells. Suitable antigens for sensitization have been described previously and thus, the cells are suitable for the treatment of infectious diseases, allergic diseases or neoplastic diseases. Thus, in another aspect, the invention relates to the antigen-presenting cells of the invention for use in the treatment of infectious diseases, allergic diseases or neoplastic diseases. In yet another aspect, the invention relates to a method for the treatment of infectious diseases, allergic diseases or neoplastic diseases comprising the administration to a patient the antigen presenting cells of the invention. In another aspect, the invention relates to the use of an antigen-presenting cell according to the invention, to be used in a method for induction of a cytotoxic cell response against the cytotoxic antigen or to be used in a method for induction of a cytotoxic cell response against an infectious, allergic or neoplastic disease.

Preferably, the methods of treatment of the present invention comprised the so-called adoptive immunotherapy. The term "adoptive immunotherapy" refers to a therapeutic approach for treating cancer or infectious diseases in which immune cells are administered to a host with the aim that the cells mediate either directly or indirectly specific immunity to (i.e., mount an immune response directed against) the undesired cells. In preferred embodiments, the immune response results in inhibition of tumor and/or metastatic cell growth and/or proliferation and most preferably results in neoplastic cell death and/or resorption The immune cells can be derived from a different organism/host (exogenous immune cells) or can be cells obtained from the subject organism (autologous immune cells)

The immune cells are typically activated *in vitro* by a particular antigen (in this case the antigenic peptide used in the conjugates of the invention) using any of the techniques mentioned above for the activation of APC in vitro. Methods of performing adoptive immunotherapy are well known to those of skill in the art (see, e g, US Pat Nos 5,081,029, 5,985,270, 5,830,464, 5,776,451, 5,229,115, 690,915, and the like). The invention contemplates numerous modalities of adoptive immunotherapy. In one embodiment, the DC (e.g. isolated from the patient or autologous dendritic cells) are pulsed with the conjugates of the invention and then injected back into the subject where they present and activate immune cells in vivo. In addition, or alternatively, the DC can be transfected with nucleic acids encoding the conjugates of the invention and then re- introduced into a patient. In yet another embodiment, the DC are pulsed with the conjugates of the invention or transfected with nucleic acids encoding the conjugates of the invention, and then used to stimulate peripheral blood lymphocytes or TIL in culture and activate CTLs targeted against the antigenic peptide that are then infused into the patient. Similarly, fibroblasts, and other APCs, or tumor cells are transfected with a nucleic acid encoding the conjugates for the invention and used to activate tumor cells or PBLs ex vivo to produce CTLs directed against the antigenic peptide that can then be infused into a patient.

Using the teachings provided herein, other therapeutic modalities utilizing the conjugates of the invention or the nucleic acids encoding said conjugates can be readily developed. As indicated above, in one embodiment the immune cells are derived from peripheral blood lymphocytes or TILs (e.g. derived from tumors/tumor suspension) Lymphocytes used for *in vitro* activation include, but are not limited to T lymphocytes, various antigen presenting cells (e g monocytes, dendritic cells, B cells, etc.) and the like. Activation can involve contacting an antigen presenting cell with the conjugates of this invention which then present the antigen (or fragment thereof), e.g. on HLA class I molecules and/or on HLA class II molecules, and/or can involve contacting a cell (e.g. T-lymphocyte) directly with the chimeric molecule. Activation of immune cells can take a number of forms including, but not limited, to the direct addition of the conjugate to peripheral blood lymphocytes (PBLs) or tumor infiltrating lymphocytes (TILs) in culture, loading of antigen presenting cells (e g monocytes, dendptic cells, etc) with the chimeric molecule in culture, transfection of antigen presenting cells, or PBLs, with a nucleic acid encoding the conjugate and the like

Inoculation of the activated cells is preferably through systemic administration. The cells can be administered intravenously through a central venous catheter or into a large peripheral vein. Other methods of administration (for example, direct infusion into an artery) are within the scope of the invention.

The dendritic cells of the invention can be provided in a formulation which is suitable for administration to a patient, e.g., intravenously. DCs of the invention that are suitable for administration to a patient are referred to herein as a "vaccine" or "DC vaccine." A vaccine or DC vaccine may further comprise additional components to help modulate the immune response, or it may be further processed in order to be suitable for administration to a patient. Methods of intravenous administration of dendritic cells are known in the art, and one of skill in the art will be able to vary the parameters of intravenous administration in order to maximize the therapeutic effect of the administered DCs.

Thus, DCs are administered to a subject in any suitable manner, often with at least one pharmaceutically acceptable carrier. The suitability of a pharmaceutically acceptable carrier is determined in part by the particular composition being administered, as well as by the particular method used to administer the composition. Most typically, quality control tests (e.g., microbiological assays clonogenic assays, viability tests), are performed and the cells are reinfused back to the subj ect, in some cases preceded by the administration of diphenhydramine and hydrocortisone. See, e.g., Korbling et al. (1986) Blood 67: 529-532 and Haas et al. (1990) Exp. Hematol. 18: 94-98.

Formulations suitable for parenteral administration, such as, for example, by intravenous administration, include aqueous isotonic sterile injection solutions which can contain antioxidants, buffers, bacteriostats, and solutes that render the formulation isotonic with the blood of the intended recipient, as well as aqueous and non-aqueous sterile suspensions that can include suspending agents, solubilizers, thickening agents, stabilizers, and preservatives. Generally, DCs of the invention can be administered to a subject at a rate determined by the effective dose, the toxicity of the cell type (e.g., the LD-50), and the side-effects of the cell type at various concentrations, as appropriate to the mass and overall health of the subject as determined by one of skill in the art. Administration can be accomplished via single or divided doses. The DCs of the invention can supplement other treatments for a disease or disorder, including, for example, conventional radiation therapy, cytotoxic agents, nucleotide analogues and biologic response modifiers.

The dose of the dendritic cells administered to a patient, in the context of the present invention should be sufficient to effect a beneficial therapeutic response in the patient over time, or to inhibit growth of cancer cells, or to inhibit infection. Thus, cells are administered to a patient in an amount sufficient to elicit an effective CTL response to the virus or tumor antigen and/or to alleviate, reduce, cure or at least partially arrest symptoms and/or complications from the disease or infection. An amount adequate to accomplish this is defined as a "therapeutically effective dose." The dose will be determined by the activity of dendritic cell produced and the condition of the patient, as well as the body weight or surface area of the patient to be treated. The size of the dose also will be determined by the existence, nature, and extent of any adverse side-effects that accompany the administration of a particular cell in a particular patient. In determining the effective amount of the cell to be administered in the treatment or prophylaxis of diseases such as cancer (e.g., metastatic melanoma, prostate cancer, etc.), the physician needs to evaluate circulating plasma levels, CTL toxicity, progression of the disease, and the induction of immune response against any introduced cell type.

Prior to infusion, blood samples are obtained and saved for analysis. Generally at least about 10⁴ to 10⁶ and typically, between 10⁸ and 10¹⁰ cells are infused intravenously or intraperitoneally into a 70 kg patient over roughly 60-120 minutes. Preferably, cell numbers of at least 10⁷ for each vaccination point are used. The injections may be e.g. 4 times repeated in a 2 weeks interval and should be given preferably near lymph nodes by intradermal or subcutaneous injections. Booster injections may be performed after a 4 weeks pause. Vital signs and oxygen saturation by pulse oximetry are closely monitored. Blood samples are obtained 5 minutes and 1 hour following infusion and saved for analysis. Cell reinfusion is repeated roughly every month for a total of 10-12 treatments in a one year period. After the first treatment, infusions can be performed on a outpatient basis at the discretion of the clinician. If the reinfusion is given as an outpatient, the participant is monitored for at least 4 hours following the therapy. For administration, cells of the present invention can be administered at a rate determined by the LD-50 (or other measure of toxicity) of the cell type, and the side-effects of the cell type at various concentrations, as applied to the mass and overall health of the patient. Administration can be accomplished via single or divided doses. In some regimens, patients may optionally receive in addition a suitable dosage of a biological response modifier including but not limited to the cytokines IFNα, IFNγ, IL-2, IL-4, IL-6, TNF or other cytokine growth factor, antisense TGFβ, antisense IL-10, and the like.

In the case that the activated cells are used to treat tumors, the cells can be used alone or in conjunction with other therapeutic regimens including but not limited to administration of IL-2, other chemotherapeutics (e.g. doxirubicin, vinblastine, vincristine, etc), radiotherapy, surgery, and the like. As indicated above, the cells may, optionally, be expanded in culture This expansion can be accomplished by repeated stimulation of the T cells with the conjugates of the invention with or without IL-2 or by growth in medium containing IL-2 alone Other methods of T cell cultivation (for example with other lymphokines, growth factors, or other bioactive molecules) are also within the scope of the invention.

### IX. METHODS FOR DELIVERY OF THERAPEUTIC ACTIVE COMPOUNDS TO CELLS EXPRESSING THE CD4, CD8, CD19, CD11C, F4/8 AND/OR CD117 MARKERS

The authors of the present invention have also observed that the PSM conjugates of the invention can efficiently bind to the surface of a large number of B lymphocytes, macrophages, CD11c dendritic cells, CD4 and CD8 T lymphocytes or of granulocytes and, in particular, are capable of binding to cells expressing CD4, CD8, CD19, CD11c, F4/8 o CD117 markers.

This finding opens the door for new additional therapeutic applications wherein a compound of interest can be delivered to cells expressing one or more of the above markers. Compounds of interest include therapeutically active compounds which are to be targeted to the specific cells as well as cytotoxic compounds which are required wherein there is an abnormally elevated number of cells or the cells show an undesired activity.

Thus, in another aspect, the invention relates to a conjugate (hereinafter "non-immunogenic conjugate) comprising
(i) a phenol soluble modulin or a functionally-equivalent variant thereof and
(ii) a biologically active compound

PSM suitable for use in the non-immunogenic conjugates of the invention have been described in detail in relation with the immunogenic conjugates.

The term "biologically active compound" is defined as a compound which is capable of preventing or eliminating the symptoms of a disease. The invention initially contemplates the use of any therapeutic compound which is susceptible to covalent modification without substantially losing its biological activity, such that it can be conjugated to PSM or to the functionally equivalent variant thereof. Thus, the invention contemplates the use of small organic molecules, peptides, peptidomimetics, peptoids, proteins, polypeptides, glycoproteins, oligosaccharides, nucleic acids and the like as a therapeutically effective component.

In a preferred embodiment, the biologically active compound is a peptidic compound. In a still more preferred embodiment, the biologically active peptidic compound forms a single polypeptide chain with the PSM. The invention also relates to polynucleotide or gene constructs comprising a sequence encoding the non-immunogenic conjugate, a vector comprising said polynucleotide or gene construct and to a host cell comprising the polynucleotide, gene construct or vector. Suitable vectors and host cells for expressing the non-immunogenic conjugates are essentially the same as those used for the immunogenic conjugates and thus, need not be further explained.

In another aspect, the invention provides a pharmaceutical composition comprising a non-immunogenic conjugate of the invention, as well as a polynucleotide coding for said conjugate, a vector or a gene construct comprising said polynucleotide or a host cell comprising said polynucleotide, vector or gene construct, together with a pharmaceutically acceptable carrier, adjuvant or vehicle for the administration to a patient. The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the formulation. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol, solutol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations such as DMSO (dimethylsulphoxide) and its derivatives.

The pharmaceutical compositions can be administered by any suitable administration route, for example an oral, topical, rectal or parenteral route (including subcutaneous, intraperitoneal, intradermal, intramuscular and intravenous route).

Suitable pharmaceutical forms for oral administration include any solid composition (tablets, pastilles, capsules, granules, etc.) or liquid composition (solutions, suspensions, emulsions, syrups, etc.) and can contain conventional excipients known in the art, such as binding agents, for example syrup, acacia, gelatin, sorbitol, tragacanth, or polyvinylpyrrolidone; fillers, for example lactose, sugar, cornstarch, calcium fosfate, sorbitol or glycine; lubricants for the preparation of tablets, for example magnesium stearate, disintegrants, for example starch, polyvinylpirrolidone, sodium starch glycolate or microcrystalline cellulose; or pharmaceutically acceptable wetting agents such as sodium laurylsulfate.

Solid oral compositions can be prepared by means of conventional methods for mixing, filling or preparing tablets. The repeated mixing operations can be used to distribute the active ingredient through the entire compositions by using large amounts of filler agents. Such operations are conventional in the art. The tablets can be prepared, for example by means of wet or dry granulation and can be optionally coated according to methods well known in normal pharmaceutical practice, particularly with an enteric coating.

The pharmaceutical compositions can also be adapted for parenteral administration such as sterile solutions, suspensions or lyophilized products in a suitable unitary pharmaceutical form. Suitable excipients such as bulk agents, buffering agents or surfactants can be used. The mentioned formulations will be prepared using usual methods such as those described or referred to in Spanish Pharmacopoeia and the Pharmacopoeia of the United States and in similar reference texts.

The administration of the compounds or compositions used in the present invention can be obtained by any suitable method, such as intravenous infusion, oral preparations and intraperitoneal and intravenous administration. Nevertheless, the preferred administration route will depend on the patient's condition. Oral administration is preferred due to the comfort for the patient and the chronic character of the diseases which are to be treated.

For their application in therapy, the compositions of the invention will preferably be found in pharmaceutically acceptable or substantially pure form, i.e. the compositions of the invention have a pharmaceutically acceptable purity level excluding the pharmaceutically acceptable excipients and not including material considered to be toxic at the normal dosage levels.

The therapeutically effective amounts of the non-immunogenic conjugates of the invention will generally depend, among other factors, on the individual who is to be treated, on the severity of the disease said individual suffers from, on the administration form chosen etc. For this reason, the doses mentioned in this invention must be considered as guides for the person skilled in the art and the latter must adjust the doses according to the variables mentioned previously. Nevertheless, the conjugates can be administered once or more times a day, for example, 1, 2, 3 or 4 times a day in a typical daily total amount comprised between 1 and 200 mg/kg body mass/day, preferably 1-10 mg/kg body mass/day. In the same manner an inhibitor of choline kinase can be administered once or more times a day, for example, 1, 2, 3 or 4 times a day in a typical daily total amount comprised between 1 and 200 mg/kg body mass/day, preferably 1-10 mg/kg body mass/day.

The non-immunogenic conjugates can be used in medicine and thus, the invention also relates to the non-immunogenic conjugates, the polynucleotides encoding said conjugates, the vector comprising said polynucleotide or gene construct, the host cell comprising the polynucleotide, gene construct or vector or the pharmaceutical compositions comprising said conjugates for use in medicine.

The non-immunogenic conjugates of the invention are particularly suited for delivery of biologically active compounds to those cell types for which the conjugates show affinity. As it has been shown in example 1, the conjugates are capable of binding to cells expressing CD4, CD8, CD11c, CD19, F480 or CD117. In those cases wherein the compound is cytotoxic or is capable of inhibiting growth suppressor genes, the compounds can be used for the treatment of diseases wherein an undesired proliferation of one or more types of the cells mentioned above occurs. However, the compounds can also be used for delivery of compounds suitable for restoring a property of any of the above mentioned cell types. The compounds may be either supplying a missing activity to the cell or may be inhibiting a gene responsible for the disappearance of a given property of the cell.

Thus, in another aspect, the invention relates to a method for the treatment of a disease characterized by an undesired proliferation or an undesired activity of a cell selected from the group of a CD4, CD8, CD11c, CD19, F480 or CD117-positive cell or a combination thereof which comprises administering to a patient the polynucleotides encoding said conjugates, the vector comprising said polynucleotide or gene construct, the host cell comprising the polynucleotide, gene construct or vector or the pharmaceutical compositions comprising said conjugates. In a preferred embodiment, the non-immunogenic conjugates comprise a cytotoxic compound and can be used for the treatment of a disease characterised by an undesired proliferation of cells expressing or CD4, CD8, CD 19, CD11c, F4/8 o CD117.

A cytotoxin or cytotoxic agent includes any agent that is detrimental to (e.g., kills) cells. For a description of these classes of drugs which are well known in the art, and their mechanisms of action, see Goodman et al., Goodman and Gilman's The Pharmacological Basis Of Therapeutics, 8th Ed., Macmillan Publishing Co., 1990. Additional techniques relevant to the preparation of antibody immunotoxins are provided in for instance Vitetta, Immunol. Today 14, 252 (1993) and US 5,194,594. Suitable therapeutic agents for forming immunoconjugates useful for the present invention include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, actinomycin D, 1-dehydro-testosterone, glucocorticoids, procaine, tetracaine, lidocaine, propranolol, and puromycin, antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DT1C), procarbazine, mitomycin C, cisplatin and other platinum derivatives, such as carboplatin), antibiotics (such as dactinomycin (formerly actinomycin), bleomycin, daunorubicin (formerly daunomycin), doxorubicin, idarubicin, mithramycin, calicheamicin, mitomycin, mitoxantrone, plicamycin, anthramycin (AMC)), diphtheria toxin and related molecules (such as diphtheria A chain and active fragments thereof and hybrid molecules), ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Therapeutic agents, which may be administered in combination with an antibody as described elsewhere herein, may also be candidates for therapeutic moieties useful for conjugation to an antibody used in the present invention. Moreover, if the cytotoxic compound is a polypeptide, this include, without limitation, an enzymatically active toxin, or active fragment thereof, such as abrin, ricin A, pseudomonas exotoxin, or diphtheria toxin; a protein such as tumor necrosis factor or interferon-gamma or, biological response modifiers such as, for example, lymphokines, interleukin-1 (IL-1 ), interleukin-2 (IL-2), interleukin-6 (IL-6), granulocyte macrophage colony stimulating factor (GM-CSF), granulocyte colony stimulating factor (G-CSF), or other growth factors and apotopic inducing protein isolated from mitochondria.

Diseases wherein it is desired to destroy CD4-positive cells are, for instance, HIV-1 infection wherein and for promoting growth inhibition of CD4-expressing cells in those situations wherein there is an increased activity of the CD4 cells against self targets or organs as it happens in autoimmune diseases such as systemic lupus erythematosis, rheumatoid arthritis, osteoarthritis, juvenile chronic arthritis, a spondyloarthropathy, systemic sclerosis, an idiopathic inflammatory myopathy, Sjogren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

Diseases characterised by an undesired proliferation or an undesired activity of a CD8-positive cell include immune-mediated diseases resulting from immune responses against self-antigens or from inappropriate responses against innocuous foreign antigens (i.e. infection with non-cytopathic viruses or with viruses mimicking self antigens).

The term "autoimmune disease", as used herein, refers to a condition in a subject characterized by cellular, tissue and/or organ injury caused by an immunologic reaction of the subject to its own cells, tissues and/or organs. Illustrative, non-limiting examples of autoimmune diseases which can be treated with the cell population of the invention include alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, sarcoidosis, scleroderma, progressive systemic sclerosis, Sjögren's syndrome, Goodpasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, Wegener's granulomatosis, etc.).

Diseases resulting from inappropriate responses against innocuous foreign antigens (i.e. infection with non-cytopathic viruses or with viruses mimicking self antigens) include HIV-1-induced neurological disease, Theiler's murine encephalomyelitis virus (TMEV) - mediated multiple sclerosis, HSV-induced myasthenia gravis, rotavirus-induced pancreatic islet autoimmunity and the like.

Diseases characterised by an undesired proliferation or an undesired activity of a cell CD11c-positive cell include any B-lineage malignancies such as lymphoid neoplasms, non-Hogdkin lymphoma, follicular lymphoma, diffuse large B cell lymphoma, chronic lymphocytic lymphoma, hairy cell leukemia, T-prolymphocytic leukemia, mantle cell lymphoma, Burkitt's type leukemia, multiple myeloma, acute lymphatic leukemia and the like.

The invention is described by way of the following examples which are to be construed as merely illustrative and not limitative of the scope of the invention.

### EXAMPLES

### Example 1. Modulin peptides are able to bind to the cell surface of the splenocytes. Analysis of the subpopulations capable of binding modulin-derived peptides.

### Material and Methods:

The peptides used in the present assays are shown in Table 3.

| Peptide | Sequence | SEQ ID NO: |
|---|---|---|
| SIINFEKL | SIINFEKL | 44 |
| αMod | MADVIAKIVEIVKGLIDQFTQK | 1 |
| αMod-SIINFEKL | MADVIAKIVEIVKGLIDQFTQKSIINFEKL | 45 |
| SIINFEKL-αMod | SIINFEKLMADVIAKIVEIVKGLIDQFTQK | 46 |
| γMod | MAADIISTIGDLVKWIIDTVNKFKK | 13 |
| γMod-SIINFEKL | MAADIISTIGDLVKWIIDTVNKFKKSIINFEKL | 47 |
| SIINFEKL-yMod | SIINFEKLMAADIISTIGDLVKWIIDTVNKFKK | 48 |
| OVA(235-264) | ASGTMSMLVLLPDEVSGLEQLESIINFEKL | 49 |
| CFSE-γMod-SIINFEKL | CFSE- MAADIISTIGDLVKWIIDTVNKFKKSIINFEKL | 47 |
| CFSE-OVA(235-264) | CFSE-ASGTMSMLVLLPDEVSGLEQLESIINFEKL | 49 |
| 1073 | CVNGVCWTV | 50 |
| αMod-1073 | MADVIAKIVEIVKGLIDQFTQKCVNGVCWTV | 51 |
| γMod-1073 | MAADIISTIGDLVKWIIDTVNKFKKCVNGVCWTV | 52 |
| δMod | MSIVSTIIEVVKTIVDIVKKFKK | 14 |
| δMod-1073 | MSIVSTIIEVVKTIVDIVKKFKKCVNGVCWTV | 53 |
| 1073-δMod | CVNGVCWTVMSIVSTIIEVVKTIVDIVKKFKK | 54 |

Binding assays were carried out using either splenocytes (Figure 1, panels A B, D, E and G) or bone marrow derived dendritic cells (Figure 1, panels C and F). The cells were fixed by means of an incubation with a 0.4% glutaraldehyde solution for 15 minutes at 4°C. 5x10⁵ fixed cells were incubated in the presence of 20 µM CFSE αMod-SIINFEKL (Figure 1, panels A, B and C), or CFSE γMod-SIINFEKL (figure 1, panels D, E, F and G),. In some assays, and to study the specificity of CFSE labeled peptide binding, these incubations were carried out in the presence or absence of the unlabeled peptides αMod-SIINFEKL, γMod-SIINFEKL or SIINFEKL (100 µM) (panels B and E). Also, we carried out binding assays using the CFSE labeled peptide CFSE-OVA(235-264) as a negative control (Figure 1, panels A, C, D and F). After 30 minutes of incubation at 4°C, two washings with PBS were carried out and the performed labeling was analyzed by flow cytometry. In some experiments (Figure 1G), double staining was carried out using anti-CD4, CD8, CD11c, CD 19, F480 or GR1(CD117) antibodies labeled with phycoerythrin (Pharmingen) together with the CFSE-γMod-SIINFEKL peptide. In these cases, the labeling with the specific antibodies was carried out before fixing the cells with glutaraldehyde. As mentioned above, in some cases, the labeling experiments with the CFSE γMod-SIINFEKL or with CFSE αMod-SIINFEKL peptide or with CFSE-OVA(235-264) control peptide were carried out on bone marrow dendritic cells obtained as indicated below (Figure 1C and 1F),.

### Results and Discussion

One of the features that can favor the activity of an immunogen to induce an efficient immune response is its capacity to be efficiently captured by antigen-presenting cells. Modulin peptides could have this capacity since it has been described that they can activate the TLR2 signaling pathway and antigen-presenting cells express this molecule ion their surface. However, there is no experimental evidence that modulin peptides bind to the surface of antigen-presenting cells. For this reason, the CFSE-γMod-SIINFEKL peptide was synthesized, containing the gamma modulin sequence bound to the SIINFEKL cytotoxic T determinant of ovoalbumin and the CFSE (Carboxyfluorescein diacetate succinimidyl ester (CFSE)) fluorescent dye was bound to the amino-terminal end of the peptide in order to analyze its capacity to bind to the cell surface by flow cytometry.

Thus, as shown in Figure 1, it was found that CFSE-αMod-SIINFEKL peptide binds to splenocytes. Indeed, splenocytes incubated with CFSE-αMod-SIINFEKL showed higher fluorescence intensity than that found on splenocytes incubated with the CFSE labeled irrelevant peptide CFSE-OVA(235-264) (Figure 1A). This positive signal was significantly inhibited by the addition of the non labeled peptide αMod-SIINFEKL but not by SIINFEKL peptide (Figure 1B), suggesting that the signal obtained with CFSE αMod-SIINFEKL is specific and related to the presence of α-Modulin. This binding capacity of CFSE αMod-SIINFEKL was also clearly observed when bone marrow derived dendritic cells were used (Figure 1C). Similar results were found when we used the CFSE-γMod-SIINFEKL peptide (Figures 1C, D and E). In this case, two fluorescence peaks (one with low fluorescence (low) and other with high fluorescence (bright) (Figure 1D,) were found. The fluorescence obtained with the CFSE labeled irrelevant peptide CFSE-OVA (235-264) was similar to the low fluorescent peak observed with CFSE γMod-SIINFEKL (Figure 1D) suggesting that this second peak was due to a non-specific binding. The incubation with the γMod-SIINFEKL peptide which does not contain CFSE and which therefore competes with the CFSE γMod-SIINFEKL peptide for the binding to its receptor, reduced the bright fluorescence (see dotted line in Figure 1E). However, the incubation with the SIINFEKL peptide did not affect this signal, indicating that the binding of the peptide to the cell surface was due to the action of the γ-modulin sequence.

As in the case of CFSE αMod-SIINFEKL peptide, the CFSE γMod-SIINFEKL peptide was also able to stain bone marrow derived dendritic cells 8Figure 1F). When double staining was carried out using anti-CD4, CD8, CD11c, CD19, F480 or GR1(CD117) antibodies labeled with phycoerythrin (Pharmingen) together with the CFSE-γMod-SIINFEKL peptide (Figure 1G), it was found that approximately 24.4 % of CD4+ cells, 17.8% of CD8+ cells, 62.1% of CD11c cells, 52.8% of CD19 cells, 66.2% of F418 cells and 27.3% of GR1+ cells were stained with the CFSE γMod-SIINFEKL peptide, indicating that γModulin binds to these cells.

This data indicates that α-modulin and γ-modulin bind specifically to some cell subtypes and in particular to antigen-presenting cells, a property that can be very useful while carrying antigens to these cells at the time of activating a specific cell response against the antigen..

### Example 2. α-modulin and γ-modulin peptides activate the maturation of dendritic cells and antigen presentation.

### Material and Methods

### Generation of dendritic cells from bone marrow.

Dendritic cells were grown from bone marrow cells. After lysing the erythrocytes with ACK lysis buffer, the cells were washed and lymphocytes and granulocytes were removed by means of incubation with a mixture of antibodies against CD4 (GK1; ATCC, Manassas, VA), CD8 (53.6.72; ATCC), Ly-6G/Gr1 (BD-Pharmingen; San Diego CA) and CD45R/B220 (BD-Pharmingen), followed by rabbit supplement. The remaining cells grew in 12 culture plates in complete medium with 10⁶ cells/ml supplemented with 20 ng/ml of mGM-CSF and 20 ng/ml of mIL-4 (both from Peprotech; London, GB). Every 2 days the medium was substituted with fresh medium containing cytokines. On day 7, non-adherent dendritic cells were collected, and cultured in the presence or absence of 50 µM of modulin peptides or of 1 µg/ml of LPS (Sigma) and incubated for 48 hours at 37°C and 5% CO2. After this period, the cells were isolated and incubated with anti IAb, CD54 and CD86 antibodies (Pharmingen). In parallel experiments, the dendritic cells were incubated with the indicated stimuli for 16 hours, after which the expression of the messenger RNA for the p40 molecules of IL-12 and of TNF-α was analyzed.

### Analysis of the expression of messenger RNA for IL-12 p40 and TNFα.

The total RNA of dendritic cells incubated for 16 hours with the different stimuli indicated above were extracted using the Nucleic Acid Purification Lysis Solution (Applied BioSystems, Foster City, CA) and the ABI PRISM 6100 Nucleic Acid PrepStation (Applied BioSystems) semiautomatic system. The treatment with DNAses, the reverse transcription and the real-time quantitative PCR for the p40 subunit ofIL-12 and TNF-alpha were carried out as previously described and using specific primers for these cytokines (Zabala, M., et to the 2007. J Hepatol 47:807-815.) The messenger RNA values were calculated using the formula: 2^{ΔCt}, where ΔCt indicates the difference in the threshold cycle between the control gene (β actin) and the genes under study.

### Upregulation of maturation markers.

Expression of DC maturation markers was measured by flow cytometry. DC were harvested and pre-incubated with a rat anti-CD16/32 mAb (2.4G2 clone, BD Pharmingen) for 15 min, to block non-specific binding of primary antibodies. After this initial incubation, cells were stained with the primary antibodies at 4°C for 15 min, washed and acquired on FACSscan cytometer (BD Biosciences, San Diego, CA) and analyzed using Cell Quest software (BD Biosciences). The antibodies used were: anti-H-2K^{b} (AF6-88.5 clone), anti-CD54 (3E2 clone), anti-CD86 (GL1 clone) and anti-CD 11c (HL-3 clone), all from BD Pharmingen.

### Antigen presentation assays.

Dendritic cells derived from bone marrow were cultured in the presence or absence of 10 µM of the indicated peptides (Figure 3). Forty hours later, the cells were washed and distributed in 96-well plates at different concentrations and incubated in the presence of 10⁵ T cells/well obtained from OT-1 transgenic mice (which express a specific T receptor for the SIINFEKL peptide of ovalbumin. Seventy-two hours later, tritiated thymidine was added to the cultures and six hours later the cells were harvested and the incorporated thymidine was analyzed in a scintillation counter (Topcount Packard).

### Results and discussion.

The maturation of dendritic cells (DC) is a requirement for the optimal stimulation of a T lymphocyte response. When maturation occurs, the APCs increase the expression of surface molecules such as MHC class I (H2Kb in this model) and class II (IAb in this model) and CD40, CD80 and CD86 molecules. Therefore, it was analyzed if the peptides derived from α-modulin or γ-modulin EDA-SIINFEKL could induce the maturation of dendritic cells derived from bone marrow. To that end, the dendritic cells were cultured as indicated in materials and methods and analyzed by flow cytometry to see the expression of these maturation markers. It can be observed in Figure 2C that the addition of α-modulin or γ-modulins induce the overexpression of some of these markers. Specifically, α-modulin activates the expression of CD54, CD86 and the IAb molecule, whereas γ-modulin promotes the expression of CD54 and the IAb molecule. This data suggests that modulins could have an enhancing effect in the activation of an immune response against an antigen by means of stimulating the maturation of dendritic cells.

When the expression of messenger RNA for IL-12 and TNF-alpha cytokines was analyzed (Figure 2A and 2B respectively), it was found that to a certain extent, α- and γ-modulins were capable of increasing the expression of these mRNAs (p<0.05 in both cases, when we compared the mRNA expression of DC cultured with α or γ- Modulins or with culture medium alone), suggesting that they are capable of promoting the secretion of these pro-inflammatory cytokines which could favor the activation of an immune response against an antigen.

Once the capacity of modulin peptides to bind to the surface of the dendritic cell and activate its maturation was analyzed, it was studied if it was capable of favoring the antigen presentation of the SIINFEKL peptide to which they were covalently bound. Thus, when the dendritic cells were incubated with 10 µM of the peptides αMod-SIINFEKL, γMod-SIINFEKL, OVA(235-264), αMod, γMod or SIINFEKL alone, and these cells were used as presenting cells to activate the proliferation of T lymphocytes of OT-1 mice (and therefore with a specific T cell receptor for the SIINFEKL peptide), it was found that T lymphocytes proliferate better when the dendritic cells have been incubated with SIINFEKL alone, which does not require antigen processing since it binds directly to MHC-class I molecules at the surface of dendritic cells. However, when we compare those peptides requiring antigen processing (longer peptides), it was found that dendritic cells incubated with αMod-SIINFEKL or with γMod-SIINFEKL stimulated OT-1 T cell proliferation significantly better than OVA(235-264) peptide (p<0.05). Thus, as it can be seen in Figure 2D, the presence of α-modulin or γ-modulin favours SIINFEKL processing and presentation by dendritic cells to SIINFEKL-specific T lymphocytes improving their proliferation.

### Example 3A. The immunization with modulin-derived peptides bound to the cytotoxic SIINFEKL antigen and in combination with some TLR ligands induce the activation of a cytotoxic response against the antigen. This cytotoxic response is long-lasting and protects the mice against the subcutaneous injection of EG.7OVA tumor cells.

### Material and Methods.

### Measurement of the in vivo induction of cytotoxic T lymphocytes (CTL) (In vivo killing) and of IFN γ producing cells after immunization (ELISPOT).

C57BL6 mice were intravenously immunized intravenously with 5 nmoles of the indicated peptides in combination with 50 µg of the TLR3 ligand poly I:C. In some cases, the peptides were immunized in the presence of TLR2 (peptidoglycan), TLR4 (LPS or the EDA protein) or TLR9 (CpG) ligand. Seven days after the immunization, the mice were intravenously injected with a mixture of 5x10⁶ splenocytes, half of which had been previously labeled with a dose of 0.25 µM C and the other half with 2.5 uM CFSE and the SIINFEKL peptide (10 µg/ml). On the following day, the mice were sacrificed and the splenocytes were analyzed by flow cytometry to quantify the number of cells with high CFSE labeling with respect to the cells with low CFSE labeling. Thus, the percentage of cell lysis of the cells incubated with the cytotoxic SIINFEKL peptide can be calculated in an experiment called in vivo killing.

For the analysis of IFNγ-producing cells specific for SIINFEKL and induced in vivo by the immunizations, ELISPOT experiments were carried out using a BD-pharmingen kit (San Diego, CA) and according to the instructions of the manufacturer. Briefly, the plates (Multiscreen HTS, Millipore, Bedford, MA) incubated overnight with the anti-IFNγ antibody were blocked for 2 hours with HL-1 medium (Biowhittaker, Verviers, Belgium) containing 10% horse serum. Thus, 5x10⁵ splenocytes were incubated in triplicate in the absence or in the presence of the antigens at 37°C and 5% CO2. On the following day, the plates were washed with PBS and incubated with biotinylated anti-IFNγ antibody (2 mg/ml). After two hours, the plates were washed and incubated with a 1/100 dilution of streptavidin-peroxidase. One hour later, the wells were washed and developed using the AEC substrate set (BD-Pharmingen). The colorimetric reaction was stopped with distilled water and the number of spots was quantified using an ELISPOT automatic reader (CTL, Aalen, Germany).

### Protection against the challenge with EG7 tumor cells expressing the OVA protein

C57BL6 mice were subcutaneously immunized with 5 nmol of the indicated peptides in combination with poly I:C. Seven days after the immunization, the mice were subcutaneously injected with 5x10⁵ or with 5x10⁷ EG7OVA cells. In some experiments, mice were first challenged s.c with 5x10⁵ cells and when tumors reached 5 mm in diameter they were treated with the indicated peptide. The tumor growth was controlled with a gage. Mice were sacrificed when the tumor reached a volume greater than 4 cm³. Kaplan-Meier plot of mice survival for each immunogen is depicted.

### Results and discussion.

As shown in Figure 3, the immunization of C57BL/6 mice with the αMod-SIINFEKL or γMod-SIINFEKL peptides is capable of inducing strong cytotoxic responses (Figure 3B) and production of IFNγ (Figure 3A) in response to the stimulation with the SIINFEKL peptide. However, the free SIINFEKL peptide is not capable of activating these responses, neither is the combination of α-modulin or γ-modulin peptides with SIINFEKL when the latter is not covalently bound to them. The capacity of these immunogens to activate a long-lasting response was also studied. When the induced response was analyzed 60 days after the immunization, it was found that α or γ-modulin-derived peptides bound to SIINFEKL were capable of inducing a specific cell response (Fig 3C, IFNγ-producing cells measured by ELISPOT and Fig 3D, cells with lysis capacity measured by *in vivo* killing) which is not capable of inducing the SIINFEKL peptide in combination with the poly I:C adjuvant. These results indicate that the covalent binding of a cytotoxic determinant to α or γ modulin peptides provides it with a special property essential for the induction of a long-lasting cytotoxic cell response against the cytotoxic antigen. This property, and according to the results observed in previous Figures, could be measured by the capacity of modulin peptides to carry the antigen towards the presenting cells. The binding of the antigen to the presenting cells could furthermore favor their maturation and the presentation of the cytotoxic determinant in a more efficient manner and this, together with the aid of an adjuvant such as poly I:C, a TLR3 ligand, could enormously enhance the activation of the cell response.

The effect of other TLR ligands in combination with modulin-derived peptides on their capacity to enhance the activation of a cytotoxic response against the SIINFEKL peptide was also studied. As shown in Figure 4, the combination of modulin peptides (αMod-SIINFEKL and γMod-SIINFEKL) with TLR3 and TLR9 ligands, and to a lesser extent with TLR4 ligands has a synergistic effect on the activation of the immune response. This data suggests that modulin peptides can be combined with these ligands for the design of potential vaccines.

Once the capacity of modulin-derived peptides to activate a cytotoxic response against the SIINFEKL antigen was demonstrated, the capacity of these immunogens to protect the mice from the injection of EG7OVA tumor cells was evaluated. Thus, mice were subcutaneously immunized with 5 nmoles of the peptides indicated in Figure 5A and 5B in combination with the poly I:C adjuvant. Seven days after the immunization, 5x10⁵ (in A) or 7x10⁵ (in B) EG7OVA cells were injected subcutaneously. It was observed that the immunization with the αMod-SIINFEKL (Figure 5A) or γMod-SIINFEKL peptides (Fig 5B) protected the mice from tumor growth. All the mice immunized with SIINFEKL or with saline developed tumors. We also tested the capacity of modulin derived peptides to treat mice bearing subcutaneous tumors. In this case, EG7OVA tumor bearing mice were treated with γMod-SIINFEKL plus poly I:C, γMod + SIINFEKL (not linked covalently) plus poly I:C, SIINFEKL plus poly I:C , plus poly I:C alone or with saline and tumor growth was evaluated. It was found that treatment of mice with γMod-SIINFEKL was able to reject the tumor in about 38% of the mice as compare to 0% for the remaining treatments (Figure 6, p<0.05) These data suggests that alpha or gamma modulin peptides can be used as carriers and adjuvants in the development of vaccination strategies against pathogens or against cancer.

Next, the immunostimulatory capacity of modulin derived peptides was tested with another antigen different from SIINFEKL in order to check whether the results could also be reproduced. The modulin derived peptides were coupled to a well characterized cytotoxic T cell determinant from the non-structural protein NS3 from hepatitis C virus (the HLA-A2-restricted NS3 peptide 1073-1081, sequence CVNGVCWTV (SEQ ID NO:50)). Thus the peptides αMod-1073, γMod-1073 and δMod-1073 as well as the peptide p1073 alone were synthesized. HHD transgenic mice (which express the human HLA_A2 molecule) were immunized with 5 nmoles of the corresponding peptide in the presence of poly I:C. Seven days after immunization, ELISPOT and in vivo killing assays were carried out as described above using target cells pulsed with peptide 1073. As it is shown in figure 7A, and 7B, the presence of α-Mod, γ-Mod as well as δ-Mod coupled to peptide 1073 allowed the induction of strong T cell immune responses specific for the peptide which are not found when mice are injected with the peptide 1073 plus poly I:C. Indeed, those mice immunized with δMod-1073, γMod-1073 and δMod-1073 had high numbers of IFNγ producing cells specific for peptide 1073 (Figure 7A) and high levels of CTL activity (Figure 7B).

In summary, this data shows that the binding of the α-modulin, γ-modulin or δ-modulin peptides to an antigen favors the carrying of the antigen towards the antigen-presenting cells, promoting their maturation and antigen presentation. This property allows these immunogens, in combination with the TLR3, 4 or 9 ligands, to induce in vivo strong cytotoxic cell responses against the antigen which may protect the mice against the growth of tumor cells which express said antigen or against cells expressing a viral antigen.

Therefore, modulin-derived peptides can form a very suitable vector for the induction of cell responses against an antigen of interest. The construction of fusion proteins based on these peptides involves a suitable strategy in vaccination protocols against tumor diseases or diseases caused by infectious agents.

### Example 3B. Immunization with peptide αMod-E7(49-57) in combination with poly I:C induces a specific cytotoxic response against peptide E7(49-57) and is capable of curing mice carrying TC1 subcutaneous tumors.

### Material and methods.

The following peptides were used in this section:

| Peptide | Sequence | SEQ ID NO: |
|---|---|---|
| E7(49-57) | RAHYNIVTF | 55 |
| αMod-E7(49-57) | MADVIAKIVEIVKGLIDQFTQKRAHYNIVTF | 56 |

### Measurement of the in vivo induction of IFN-γ after immunization (ELISPOT).

C57BL6 mice were intravenously immunized with 5 nmoles of the indicated peptides in combination with 50 µg of the TLR3 ligand poly I:C. For the analysis of the IFN-γ producing cells in the presence of the peptide E7(49-57) and induced *in vivo* by the immunizations, the animals were sacrificed 7 days after the immunizations and ELISPOT experiments were conducted using a BD-pharmingen kit (San Diego, CA) as indicated above and using peptide E7(49-57) as an antigen.

### Protection against the challenge with TC1 tumor cells expressing the E7 protein of the HPV16 virus (human papillomavirus 16)

The C57B/6 mice were subcutaneously injected with 5 x 10⁵ TC-1 tumor cells (expressing the E7 protein of the HPV-16 virus). After 25 days, when the tumor reaches a diameter of 8 mm, the mice are intravenously treated with PBS (B), with peptide E7(49-57) plus 50 µg/ml poly I:C (C), or with peptide αMod-E7(49-57) plus 50 µg/ml poly I:C (D). Seven days later, the mice received a second immunization of the same immunogens. The tumor size, presented as the average of two perpendicular diameters, was measured at regular intervals using a gauge. The number of tumor-free mice relative to the total number of animals per group is included for each treatment.

### Results and discussion.

In the previous experiments it was demonstrated that modulin-derived peptides were capable of binding to the surface of the splenocytes, activating the maturation of antigen-presenting cells and acting as a carrier for transporting antigens, such that the immunization with fusion peptides between these modulin-derived peptides and a cytotoxic T determinant such as SIINFEKL in combination with a TLR ligand was capable of inducing strong cytotoxic T responses *in vivo* and of protecting mice after the challenge of a EG7OVA tumor cell expressing the antigen. The intention was to see if this therapeutic effect observed in the EG7OVA tumor model could be extrapolated to other tumor models.

Cervical cancer is one of the most common cancers in women all over the world, and the fifth most frequent cancer in general, with an estimated prevalence of 1.4 million cases. There is consistent evidence that chronic genital tract infection due to various types of mucosatropic human papillomaviruses (HPV) causes cervical cancer. It has thus been postulated that HPV acts as a cervical carcinogenesis initiator and that the malignant transformation depends on the interaction with other factors.

The currently used vaccine against uterine cancer of the United States Merck laboratory, called Gardasil is indicated for the prevention of the infection by HPV-16 and HPV-17 virus strains which are in the origin of approximately 70 percent of all uterine cancer cases. This vaccine has proved to be effective in the prevention of this disease when it is administered to girls at very early ages (11-12 years). However, this vaccine is not indicated for the treatment if uterine cancer, once the latter has been manifested. The expression of the oncogenic proteins of HPV E6 and E7 is necessary for the start and the maintenance of malignant transformation and the cell immunity to E7 which have been associated with the clinical and cytological resolution of HPV-induced lesions. For this reason, the TC1 tumor model, which expresses HPV16 E7 antigen as a tumor antigen, has been used. Once the tumors have been established, reaching a diameter of 8 mm after the subcutaneous injection of the tumor cells, the animals were vaccinated with the different alternatives. It was found that those animals which received the immunization with peptide αMod-E7(49-57) and poly I:C eliminate the tumor completely. Thus, 11 of 11 mice treated with this immunogen are cured, whereas when the mice are treated with peptide E7(49-57), only 4 of 11 mice achieve the elimination of the tumor. These data suggest that the fusion peptide αMod-E7(49-57), or similar peptides based on the fusion between modulin-derived peptides and an HPV antigen can be considered for the development of an alternative therapy against human cervical carcinoma.

### Example 4. The capacity of modulin-derived peptides to induce a cell response against the antigen is independent of the activation of the TLR2 pathway.

### Material and Methods.

### Measurement of the in vivo induction of cytotoxic T lymphocytes (CTL) (In vivo killing) and of IFNγ-producing cells after immunization (ELISPOT) in KO mice for TLR2.

C57BL6 wild type mice and knockout mice were immunized for TLR2 with the aim of studying the dependence on TLR2 in the immunostimulating capacity of modulin-derived peptides. Thus, different mice were injected with 5 nmoles of the indicated peptides or with PBS in combination with 50 µg of the TLR3 ligand poly I:C. Seven days after the immunization, the mice were intravenously injected with a mixture of 5x10⁶ splenocytes, half of which had been previously labeled with a dose of 0.25 µM C and the other half with 2.5 µM CFSE and the SIINFEKL peptide (10 µg/ml). On the following day, the mice were sacrificed and the splenocytes were analyzed by flow cytometry to quantify the number of cells with high CFSE labeling with respect to the cells with low CFSE labeling in the same manner as in Example 3.

For the analysis of IFNγ-producing cells in the presence of SIINFEKL and induced in vivo by the immunizations, ELISPOT experiments were carried out using a BD-pharmingen kit (San Diego, CA) and according to the instructions of the manufacturer, in the same manner as in Example 3.

### Binding assays

Mouse splenocytes were purified from C57BL/6 TLR2 KO mice after homogenizing the spleen. The cells were fixed by means of an incubation with a 0.4% glutaraldehyde solution for 15 minutes at 4°C. 5x10⁵ fixed splenocytes were incubated in the presence of 20 µM CFSE γMod-SIINFEKL, CFSE αMod-SIINFEKL or CFSE-OVA(235-264) control peptide to show the binding specificity. After 30 minutes of incubation at 4°C, two washings with PBS were carried out and the performed labeling was analyzed by flow cytometry.

### Results and discussion.

As expected, the immunization with the αMod-SIINFEKL peptides or with γMod-SIINFEKL is capable of inducing cytotoxic T responses against target cells incubated with the SIINFEKL peptide when C57BL/6 wild type mice are immunized. In the same way, the activation of IFN-γ-producing cells specific for the SIINFEKL peptide (Figure 8B and 8A respectively) is induced. However, this response is surprisingly also observed when mice lacking the molecule TLR2 are immunized. Indeed, the response level found is similar in both cases of mice, suggesting that the TLR2 molecule is not responsible for the capacity of the modulin-derived peptides used to favor the induction of cell responses against the SIINFEKL T determinant (Figure 8).

Accordingly, when the binding assays using CFSE labeled peptide were carried out, it was found that both CFSE αMod-SIINFEKL and CFSE γMod-SIINFEKL peptides still retained the capacity to bind to the surface of splenocytes (Figure 9) suggesting that this binding is not related to TLR2 molecule.

It can be concluded with these experiments that although it has not been described in the literature that modulin peptides activate the TLR2 pathway, this property, if is correct, is not necessary for their immunoenhancing capacity. The mechanism of action of these peptides to favor the induction of cell responses against the antigen to which they are bound is still to be discovered.

SEQUENCE LISTING

<110>  PROYECTO DE BIOMEDICINA CIMA, S.L.
       INSTITUT PASTEUR

<120>  USE OF PHENOL SOLUBLE MODULINS FOR THE DEVELOPMENT OF VACCINES

<130>  P4150PC00

<150>  P200803441.0
<151>  2008-12-03

<160>  56

<170>  PatentIn version 3.5

<210>  1
<211>  22
<212>  PRT
<213>  Staphylococcus epidermidis RP62A

<400>  1

Met Ala Asp Val Ile Ala Lys Ile Val Glu Ile Val Lys Gly Leu Ile
1               5                   10                  15

Asp Gln Phe Thr Gln Lys
            20

<210>  2
<211>  44
<212>  PRT
<213>  Staphylococcus epidermidis RP62A

<400>  2

Met Ser Lys Leu Ala Glu Ala Ile Ala Asn Thr Val Lys Ala Ala Gln
1               5                   10                  15

Asp Gln Asp Trp Thr Lys Leu Gly Thr Ser Ile Val Asp Ile Val Glu
            20                  25                  30

Ser Gly Val Ser Val Leu Gly Lys Ile Phe Gly Phe
        35                  40

<210>  3
<211>  44
<212>  PRT
<213>  Staphylococcus epidermidis RP62A

<400>  3

Met Glu Gln Leu Phe Asp Ala Ile Arg Ser Val Val Asp Ala Gly Ile
1               5                   10                  15

Asn Gln Asp Trp Ser Gln Leu Ala Ser Gly Ile Ala Gly Ile Val Glu
            20                  25                  30

Asn Gly Ile Ser Val Ile Ser Lys Leu Leu Gly Gln
        35                  40

<210>  4
<211>  54
<212>  PRT
<213>  Staphylococcus epidermidis R?62A

<400>  4

Met Glu Leu Leu Thr His Leu Gly Val Leu Ile Met Lys Leu Phe Asn
1               5                   10                  15

Ala Phe Lys Asp Ile Leu Glu Ala Ala Ile Thr Asn Asp Gly Thr Gln
            20                  25                  30

Leu Gly Ala Ser Ile Val Asn Ile Ile Glu Ser Ser Val Asp Met Val
        35                  40                  45

Asn Arg Phe Leu Gly Asn
    50

<210>  5
<211>  50
<212>  PRT
<213>  Staphylococcus epidermidis R?62A

<400>  5

Met Glu His Val Ser Lys Leu Ala Glu Ala Ile Ala Asn Thr Val Ser
1               5                   10                  15

Ala Ala Gln Ala Glu Asp Gly Ala Glu Leu Ala Lys Ser Ile Val Asn
            20                  25                  30

Ile Val Ala Asn Ala Gly Gly Ile Ile Gln Asp Ile Ala His Ala Phe
        35                  40                  45

Gly Tyr

    50

<210>  6
<211>  50
<212>  PRT
<213>  Staphylococcus epidermidis RP62A

<400>  6

Met Glu His Val Ser Lys Leu Gly Glu Ala Ile Val Asp Thr Val Thr
1               5                   10                  15

Ala Ala Gln Ala Glu Asp Gly Ala Glu Leu Ala Lys Ser Ile Val Asn
            20                  25                  30

Ile Val Ala Asn Ala Gly Gly Ile Ile Gln Asp Ile Ala His Ala Phe
        35                  40                  45

Gly Tyr
    50

<210>  7
<211>  44
<212>  PRT
<213>  Staphylococcus aureus

<400>  7

Met Thr Gly Leu Ala Glu Ala Ile Ala Asn Thr Val Gln Ala Ala Gln
1               5                   10                  15

Gln His Asp Ser Val Lys Leu Gly Thr Ser Ile Val Asp Ile Val Ala
            20                  25                  30

Asn Gly Val Gly Leu Leu Gly Lys Leu Phe Gly Phe
        35                  40

<210>  8
<211>  44
<212>  PRT
<213>  Staphylococcus aureus

<400>  8

Met Glu Gly Leu Phe Asn Ala Ile Lys Asp Thr Val Thr Ala Ala Ile
1               5                   10                  15

Asn Asn Asp Gly Ala Lys Leu Gly Thr Ser Ile Val Ser Ile Val Glu
            20                  25                  30

Asn Gly Val Gly Leu Leu Gly Lys Leu Phe Gly Phe
        35                  40

<210>  9
<211>  20
<212>  PRT
<213>  Staphylococcus aureus

<400>  9

Met Ala Ile Val Gly Thr Ile Ile Lys Ile Ile Lys Ala Ile Ile Asp
1               5                   10                  15

Ile Phe Ala Lys
            20

<210>  10
<211>  22
<212>  PRT
<213>  Staphylococcus aureus

<400>  10

Met Glu Phe Val Ala Lys Leu Phe Lys Phe Phe Lys Asp Leu Leu Gly
1               5                   10                  15

Lys Phe Leu Gly Asn Asn
            20

<210>  11
<211>  21
<212>  PRT
<213>  Staphylococcus aureus

<400>  11

Met Gly Ile Ile Ala Gly Ile Ile Lys Phe Ile Lys Gly Leu Ile Glu
1               5                   10                  15

Lys Phe Thr Gly Lys
            20

<210>  12
<211>  21
<212>  PRT

<213>  Staphylococcus aureus

<400>  12

Met Gly Ile Ile Ala Gly Ile Ile Lys Phe Ile Lys Gly Leu Ile Glu
1               5                   10                  15

Lys Phe Thr Gly Lys
            20

<210>  13
<211>  25
<212>  PRT
<213>  Staphylococcus epidermidis

<400>  13

Met Ala Ala Asp Ile Ile Ser Thr Ile Gly Asp Leu Val Lys Trp Ile
1               5                   10                  15

Ile Asp Thr Val Asn Lys Phe Lys Lys
            20                  25

<210>  14
<211>  23
<212>  PRT
<213>  Staphylococcus aureus

<400>  14

Met Ser Ile Val Ser Thr Ile Ile Glu Val Val Lys Thr Ile Val Asp
1               5                   10                  15

Ile Val Lys Lys Phe Lys Lys
            20

<210>  15
<211>  25
<212>  PRT
<213>  Staphylococcus aureus

<400>  15

Met Ala Gln Asp Ile Ile Ser Thr Ile Gly Asp Leu Val Lys Trp Ile
1               5                   10                  15

Ile Asp Thr Val Asn Lys Phe Thr Lys
            20                  25

<210>  16
<211>  25
<212>  PRT
<213>  Staphylocoocus aureus

<400>  16

Met Ala Gln Asp Ile Ile Ser Thr Ile Ser Asp Leu Val Lys Trp Ile
1               5                   10                  15

Ile Asp Thr Val Asn Lys Phe Thr Lys
            20                  25

<210>  17
<211>  23
<212>  PRT
<213>  Staphylococcus intermedius

<400>  17

Met Ala Gly Asp Ile Val Gly Thr Ile Gly Glu Phe Val Lys Leu Ile
1               5                   10                  15

Ile Glu Thr Val Gln Lys Phe
            20

<210>  18
<211>  44
<212>  PRT
<213>  Staphylococcus hemolyticus

<400>  18

Met Gln Lys Leu Ala Glu Ala Ile Ala Ala Ala Val Gln Ala Gly Gln
1               5                   10                  15

Asp Lys Asp Trp Gly Lys Met Gly Thr Ser Ile Val Gly Ile Val Glu
            20                  25                  30

Asn Gly Ile Ser Val Leu Gly Lys Ile Phe Gly Phe
        35                  40

<210>  19
<211>  44
<212>  PRT
<213>  Staphylococcus hemolyticus

<400>  19

Met Glu Lys Ile Ala Asn Ala Val Lys Ser Ala Ile Glu Ala Gly Gln
1               5                   10                  15

Asn Gln Asp Trp Thr Lys Leu Gly Thr Ser Ile Leu Asp Ile Val Ser
            20                  25                  30

Asn Gly Val Thr Glu Leu Ser Lys Ile Phe Gly Phe
        35                  40

<210>  20
<211>  44
<212>  PRT
<213>  Staphylococcus hemolyticus

<400>  20

Met Ser Lys Leu Val Gln Ala Ile Ser Asp Ala Val Gln Ala Gln Gln
1               5                   10                  15

Asn Gln Asp Trp Ala Lys Leu Gly Thr Ser Ile Val Gly Ile Val Glu
            20                  25                  30

Asn Gly Val Gly Ile Leu Gly Lys Leu Phe Gly Phe
        35                  40

<210>  21
<211>  43
<212>  PRT
<213>  Staphylococcus lugdunensis

<400>  21

Met Ser Gly Ile Val Asp Ala Ile Thr Lys Ala Val Gln Ala Gly Leu
1               5                   10                  15

Asp Lys Asp Trp Ala Thr Met Ala Thr Ser Ile Ala Asp Ala Ile Ala
            20                  25                  30

Lys Gly Val Asp Phe Ile Ala Gly Phe Phe Asn
        35                  40

<210>  22
<211>  43
<212>  PRT
<213>  Staphylococcus lugdunensis

<400>  22

Met Ser Gly Ile Ile Glu Ala Ile Thr Lys Ala Val Gln Ala Gly Leu
1               5                   10                  15

Asp Lys Asp Trp Ala Thr Met Gly Thr Ser Ile Ala Glu Ala Leu Ala
            20                  25                  30

Lys Gly Ile Asp Ala Ile Ser Gly Leu Phe Gly
        35                  40

<210>  23
<211>  43
<212>  PRT
<213>  Staphylococcus lugdunensis

<400>  23

Met Asp Gly Ile Phe Glu Ala Ile Ser Lys Ala Val Gln Ala Gly Leu
1               5                   10                  15

Gln Lys Asp Trp Ala Thr Met Gly Thr Ser Ile Ala Glu Ala Leu Ala
            20                  25                  30

Lys Gly Val Asp Phe Ile Ile Gly Leu Gly His
        35                  40

<210>  24
<211>  23
<212>  PRT
<213>  Staphylococcus warneri

<400>  24

Met Ala Gly Asp Ile Val Gly Thr Ile Gly Glu Phe Val Lys Leu Ile
1               5                   10                  15

Ile Glu Thr Val Gln Lys Phe
            20

<210>  25
<211>  25
<212>  PRT
<213>  Staphylococcus warneri

<400>  25

Met Ala Ala Asp Ile Ile Ser Thr Ile Gly Asp Leu Val Lys Leu Ile

1               5                   10                  15


Ile Asn Thr Val Lys Lys Phe Gln Lys
            20                  25



<210>  26
<211>  25
<212>  PRT
<213>  Staphyloccus warneri

<400>  26

Met Thr Ala Asp Ile Ile Ser Thr Ile Gly Asp Phe Val Lys Trp Ile
1               5                   10                  15


Leu Asp Thr Val Lys Lys Phe Thr Lys
            20                  25



<210>  27
<211>  14
<212>  PRT
<213>  Artificial

<220>
<223>  Linker

<400>  27

Ser Gly Gly Thr Ser Gly Ser Thr Ser Gly Thr Gly Ser Thr
1               5                   10


<210>  28
<211>  15
<212>  PRT
<213>  Artificial

<220>
<223>  Linker

<400>  28

Ala Gly Ser Ser Thr Gly Ser Ser Thr Gly Pro Gly Ser Thr Thr
1               5                   10                  15


<210>  29
<211>  7
<212>  PRT
<213>  Artificial

<220>

<223>  Linker

<400>  29

Gly Gly Ser Gly Gly Ala Pro
1               5


<210>  30
<211>  8
<212>  PRT
<213>  Artificial

<220>
<223>  Linker

<400>  30

Gly Gly Gly Val Glu Gly Gly Gly
1               5


<210>  31
<211>  7
<212>  PRT
<213>  Artificial

<220>
<223>  Linker

<400>  31

Gly Thr Lys Val His Met Lys
1               5


<210>  32
<211>  13
<212>  PRT
<213>  Artificial

<220>
<223>  Linker

<400>  32

Pro Gly Thr Ser Gly Gln Gln Pro Ser Val Gly Gln Gln
1               5                   10


<210>  33
<211>  5
<212>  PRT
<213>  Artificial

<220>

<223>  Linker

<400>  33

Gly Thr Ser Gly Gln
1               5


<210>  34
<211>  10
<212>  PRT
<213>  Artificial

<220>
<223>  Linker

<400>  34

Pro Lys Pro Ser Thr Pro Pro Gly Ser Ser
1               5                   10


<210>  35
<211>  11
<212>  PRT
<213>  Artificial

<220>
<223>  Linker

<400>  35

Ala Pro Ala Glu Thr Lys Ala Glu Pro Met Thr
1               5                   10


<210>  36
<211>  5
<212>  PRT
<213>  Artificial

<220>
<223>  Enterokinase cleavage site

<400>  36

Asp Asp Asp Asp Lys
1               5


<210>  37
<211>  5
<212>  PRT
<213>  Artificial

<220>

<223>  Factor Xa cleavage site

<400>  37

Ile Glu Asp Gly Arg
1               5


<210>  38
<211>  6
<212>  PRT
<213>  Artificial

<220>
<223>  Thrombin cleavage site

<400>  38

Leu Val Pro Arg Gly Ser
1               5


<210>  39
<211>  7
<212>  PRT
<213>  Artificial

<220>
<223>  TEV Protease cleavage site

<400>  39

Glu Asn Leu Tyr Phe Gln Gly
1               5


<210>  40
<211>  8
<212>  PRT
<213>  Artificial

<220>
<223>  PreScission protease cleavage site

<400>  40

Leu Glu Val Leu Phe Gln Gly Pro
1               5


<210>  41
<211>  5
<212>  PRT
<213>  Artificial

<220>

<223>  MMP-9 cleavage site

<400>  41

Leu Phe Pro Thr Ser
1               5


<210>  42
<211>  45
<212>  PRT
<213>  Artificial

<220>
<223>  PSM fusion

<400>  42

Leu Met Ser Cys Leu Ile Leu Arg Ile Phe Ile Leu Ile Lys Glu Gly
1               5                   10                  15


Val Ile Ser Met Ala Gln Asp Ile Ile Ser Thr Ile Gly Asp Leu Val
            20                  25                  30


Lys Trp Ile Ile Asp Thr Val Asn Lys Phe Thr Lys Lys
        35                  40                  45


<210>  43
<211>  36
<212>  PRT
<213>  Artificial

<220>
<223>  PSM fusion

<400>  43

Met Ala Gln Asp Ile Ile Ser Thr Ile Gly Asp Leu Val Lys Trp Ile
1               5                   10                  15


Ile Asp Thr Val Asn Lys Phe Thr Lys Lys Lys Lys Lys Lys Lys Lys
            20                  25                  30


Lys Lys Lys Lys
        35


<210>  44
<211>  8
<212>  PRT
<213>  Artificial

<220>
<223>  SIINFEKL sequence

<400>  44

Ser Ile Ile Asn Phe Glu Lys Leu
1               5


<210>  45
<211>  30
<212>  PRT
<213>  Artificial

<220>
<223>  alpha-Modulin - SIINFEKL fusion

<400>  45

Met Ala Asp Val Ile Ala Lys Ile Val Glu Ile Val Lys Gly Leu Ile
1               5                   10                  15


Asp Gln Phe Thr Gln Lys Ser Ile Ile Asn Phe Glu Lys Leu
            20                  25                  30


<210>  46
<211>  30
<212>  PRT
<213>  Artificial

<220>
<223>  SIINFEKL - alphaMod fusion

<400>  46

Ser Ile Ile Asn Phe Glu Lys Leu Met Ala Asp Val Ile Ala Lys Ile
1               5                   10                  15


Val Glu Ile Val Lys Gly Leu Ile Asp Gln Phe Thr Gln Lys
            20                  25                  30


<210>  47
<211>  33
<212>  PRT
<213>  Artificial

<220>
<223>  gammaMod - SIINFEKL fusion

<400>  47

Met Ala Ala Asp Ile Ile Ser Thr Ile Gly Asp Leu Val Lys Trp Ile
1               5                   10                  15

Ile Asp Thr Val Asn Lys Phe Lys Lys Ser Ile Ile Asn Phe Glu Lys
            20                  25                  30

Leu

<210>  48
<211>  33
<212>  PRT
<213>  Artificial

<220>
<223>  SIINFEKL - gammaMod fusion

<400>  48

Ser Ile Ile Asn Phe Glu Lys Leu Met Ala Ala Asp Ile Ile Ser Thr
1               5                   10                  15

Ile Gly Asp Leu Val Lys Trp Ile Ile Asp Thr Val Asn Lys Phe Lys
            20                  25                  30

Lys

<210>  49
<211>  30
<212>  PRT
<213>  Artificial

<220>
<223>  235-264 fragment of ovalbumin

<400>  49

Ala Ser Gly Thr Met Ser Met Leu Val Leu Leu Pro Asp Glu Val Ser
1               5                   10                  15

Gly Leu Glu Gln Leu Glu Ser Ile Ile Asn Phe Glu Lys Leu
            20                  25                  30

<210>  50
<211>  9
<212>  PRT
<213>  Artificial

<220>
<223>  1073-1081 region of NS3 protein from HCV

<400>  50

Cys Val Asn Gly Val Cys Trp Thr Val
1               5


<210>  51
<211>  31
<212>  PRT
<213>  Artificial

<220>
<223>  Fusion of 1073 peptide to alpha-modulin

<400>  51

Met Ala Asp Val Ile Ala Lys Ile Val Glu Ile Val Lys Gly Leu Ile
1               5                   10                  15


Asp Gln Phe Thr Gln Lys Cys Val Asn Gly Val Cys Trp Thr Val
            20                  25                  30


<210>  52
<211>  34
<212>  PRT
<213>  Artificial

<220>
<223>  Fusion of 1073 peptide to gamma modulin

<400>  52

Met Ala Ala Asp Ile Ile Ser Thr Ile Gly Asp Leu Val Lys Trp Ile
1               5                   10                  15


Ile Asp Thr Val Asn Lys Phe Lys Lys Cys Val Asn Gly Val Cys Trp
            20                  25                  30


Thr Val


<210>  53
<211>  32
<212>  PRT
<213>  Artificial

<220>

<223>  Fusion of 1073 peptide to delta-modulin

<400>  53

Met Ser Ile Val Ser Thr Ile Ile Glu Val Val Lys Thr Ile Val Asp
1               5                   10                  15

Ile Val Lys Lys Phe Lys Lys Cys Val Asn Gly Val Cys Trp Thr Val
            20                  25                  30

<210>  54
<211>  32
<212>  PRT
<213>  Artificial

<220>
<223>  Fusion of 1073 peptide to delta-modulin

<400>  54

Cys Val Asn Gly Val Cys Trp Thr Val Met Ser Ile Val Ser Thr Ile
1               5                   10                  15

Ile Glu Val Val Lys Thr Ile Val Asp Ile Val Lys Lys Phe Lys Lys
            20                  25                  30

<210>  55
<211>  9
<212>  PRT
<213>  Artificial

<220>
<223>   Amino acids 49-57 of HPV16 E7 protein

<400>  55

Arg Ala His Tyr Asn Ile Val Thr Phe
1               5

<210>  56
<211>  31
<212>  PRT
<213>  Artificial

<220>
<223>  Fusion of alpha-modulin to amino acids 49-57 of HPV16 E7 pro

<400>  56

Met Ala Asp Val Ile Ala Lys Ile Val Glu Ile Val Lys Gly Leu Ile
1               5                   10                  15

Asp Gln Phe Thr Gln Lys Arg Ala His Tyr Asn Ile Val Thr Phe
            20                  25                  30

## Claims

1. A conjugate comprising
(i) a phenol-soluble modulin (PSM) or a functionally-equivalent variant thereof and
(ii) one or more antigenic peptides
wherein components (i) and (ii) are covalently coupled and wherein the conjugate promotes a cytotoxic response towards the antigenic peptide or peptides.

2. A conjugate according to claim 1 wherein the PSM is selected from the group of SEQ ID NO:1 to 10 or a functionally-equivalent variant thereof.

3. A conjugate according to claim 1 or 2 wherein component (ii) forms a single polypeptide chain with component (i).

4. A polynucleotide or gene construct comprising a nucleic acid sequence encoding a conjugate as defined in claim 3.

5. A vector comprising a polynucleotide or gene construct as defined in 4.

6. A host cell comprising a polynucleotide or gene construct as defined in 4 or a vector as defined in 5.

7. A composition comprising, together or separately,
(a) A conjugate as defined in claims 1 to 3, a polynucleotide or gene construct as defined in claim 4, a vector as defined in claim 5, a host cell as defined in claim 6 and
(b) a second component selected from the group of
(i) one or more toll-like receptor agonists,
(ii) one or more agonist antibodies of a co-stimulatory molecule,
(iii) one of more cytokines and
(iv) any combination of the compounds mentioned in (i) to (iii)

8. A composition according to claim 7 wherein the second toll-like receptor agonist is selected from the group of a TLR3 ligand, a TLR4 ligand and a TLR9 ligand.

9. A composition according to claim 8 wherein the TLR3 ligand is poly I:C, the TLR4 ligand is LPS or the EDA protein and/or the TLR9 ligand is CpG.

10. A method for obtaining an antigen-primed antigen-presenting cell comprising the steps of
(i) contacting an antigen-presenting cell with a conjugate as defined in claims 1 to 3, a polynucleotide or gene construct as defined in claim 4, a vector as defined in claim 5, a host cell as defined in claim 6 or a composition as defined in any of claims 7 to 9 and
(ii) isolating the antigen-primed antigen-presenting cell.

11. A method as defined in claim 10 wherein the antigen-presenting cell is a dendritic cell.

12. An antigen presenting cell obtained by the methods as defined in claims 10 or 11.

13. A pharmaceutical preparation or a vaccine comprising a conjugate as defined in claims 1 to 3, a polyucleotide or gene construct as defined in claim 4, a vector as defined in claim 5, a host cell as defined in claim 6, a composition as defined in any of claims 7 to 9 or an antigen presenting cell as defined in claim 12 and a pharmaceutically acceptable carrier.

14. A conjugate as defined in claims 1 to 3, a polyucleotide or gene construct as defined in claim 4, a vector as defined in claim 5, a host cell as defined in claim 6, a composition as defined in any of claims 7 to 9 , a pharmaceutical composition or vaccine as defined in claim 10 or an antigen presenting cell as defined in claim 12 for use in medicine.

15. A conjugate as defined in claims 1 to 3, a polyucleotide or gene construct as defined in claim 4, a vector as defined in claim 5, a host cell as defined in claim 6, a composition as defined in any of claims 7 to 9, a pharmaceutical composition as defined in claim 10 or an antigen presenting cell as defined in claim 12 for use in a method of inducing a cytotoxic response towards the antigenic peptide.

16. A conjugate as defined in claims 1 to 3, a polyucleotide or gene construct as defined in claim 4, a vector as defined in claim 5, a host cell as defined in claim 6, a composition as defined in any of claims 7 to 9, a pharmaceutical composition as defined in claim 10 or an antigen presenting cell as defined in claim 12 for use in a method of inducing a cytotoxic response towards an infectious disease, an allergic disease or a neoplastic disease.

17. Use of a conjugate as defined in claims 1 to 3, a polyucleotide or gene construct as defined in claim 4, a vector as defined in claim 5, a host cell as defined in claim 6, a composition as defined in any of claims 7 to 9 in an *in vitro* method for promoting the presentation of the antigenic peptide or peptides by antigen-presenting cells or for promoting maturation of antigen-presenting cells.

18. A conjugate comprising
(i) a phenol-soluble modulin (PSM) or a functionally-equivalent variant thereof and
(ii) a biologically active compound
wherein components (i) and (ii) are covalently coupled.

19. A conjugate according to claim 18 wherein the PSM is selected from the group of SEQ ID NO: 1 to 10 or a functionally-equivalent variant thereof.

20. A conjugate according to claims 18 or 19 wherein component (ii) is a peptidic compound which forms a single polypeptide chain with component (i).

21. A polynucleotide or gene construct comprising a nucleic acid sequence encoding a conjugate as defined in claim 20.

22. A vector comprising a polynucleotide or gene construct as defined in claim 21.

23. A host cell comprising a polynucleotide or gene construct as defined in 22.

24. A pharmaceutical preparation comprising a conjugate as defined in claim 18 to 20, a polynucleotide or gene construct as defined in claim 21, a vector as defined in claim 22 or a host cell as defined in claim 23 and a pharmaceutically acceptable carrier.

25. A conjugate as defined in claim 18 to 20, a polynucleotide or gene construct as defined in claim 21, a vector as defined in claim 22 or a host cell as defined in claim 23 or a pharmaceutical preparation as defined in claim 24 for use in medicine.

26. A conjugate as defined in claim 18 to 20, a polynucleotide or gene construct as defined in claim 21, a vector as defined in claim 22 or a host cell as defined in claim 23 or a pharmaceutical preparation as defined in claim 24 wherein the for use in the treatment of a disease **characterized by** an undesired proliferation or an undesired activity of a cell selected from the group of a CD4-, CD8-, CD19-, CD11c-, F4/8- or a CD 117-positive cells or a combination thereof.

27. A conjugate as defined in claim 18 to 20, a polynucleotide or gene construct as defined in claim 21, a vector as defined in claim 22 or a host cell as defined in claim 23 or a pharmaceutical preparation as defined in claim 24 wherein the disease **characterized by** an undesired proliferation or undesired activity of a cell selected from the group of a CD4-, CD8-, CD 19-, CD11c-, F4/8- or a CD117-positive cell is an autoimmune disease, a retroviral infection of a CD4-positive cell, a disease resulting from an immune response against a self-antigen induced by an innocuous foreign agent and a B-lineage malignancy.
